(19) Europäisches Patentamt
European Patent Office
Office européen des brevets

(11) Numéro de publication: **0 394 135**
**A1**

# (12) DEMANDE DE BREVET EUROPEEN

(21) Numéro de dépôt: **90401067.5**

(51) Int. Cl.5: **C07H 17/08, A61K 31/71**

(22) Date de dépôt: **20.04.90**

(30) Priorité: **20.04.89 FR 8905246**

(43) Date de publication de la demande: **24.10.90 Bulletin 90/43**

(84) Etats contractants désignés: **AT BE CH DE DK ES FR GB GR IT LI LU NL SE**

(71) Demandeur: **ADIR ET COMPAGNIE**
**1 rue Carle Hébert**
**F-92415 Courbevoie Cédex(FR)**

(72) Inventeur: **Lukacs, Gabor**
**22 boulevard Kellermann**
**F-75013 Paris(FR)**
Inventeur: **Duchatelle-Ruggeri, Catherine**
**1 place Jacques Froment**
**F-75018 Paris(FR)**
Inventeur: **Olesker, Alain**
**Résidence "Les Fonds Fanettes", Bâtiment 7**
**F-91190 Gif-Sur-Yvette(FR)**
Inventeur: **Ming, Li**
**Chez Mme Prevost, 73 rue du Général Leclerc**
**F-91190 Gif-Sur-Yvette(FR)**
Inventeur: **Bobillot, Sylvie**
**44 rue de la Route de Paris**
**F-78460 Chevreuse(FR)**
Inventeur: **Thatthang, Ton**
**604 rue de l'Aiguelongue**
**F-34000 Montpellier(FR)**

## (54) Noveaux dérivés macrolides, leur procédé de préparation et leur compositions pharmaceutiques qui les contiennent.

(57) Composés de la formule (I)

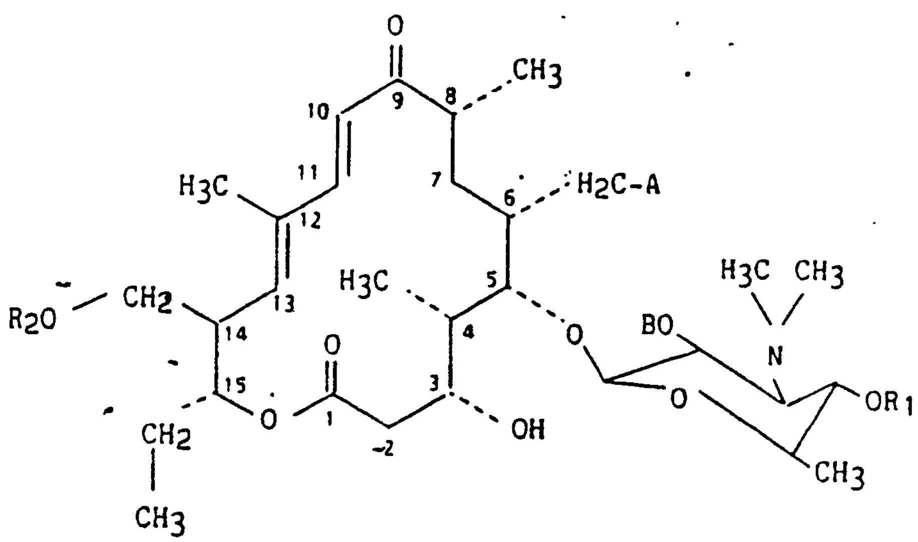

(I)

dans laquelle :
- B représente un atome d'hydrogène ou un groupement acyle inférieur,
- A représente :
- un groupement $CH_2OH$ et dans ce cas B représente un groupement acyle inférieur,

EP 0 394 135 A1

- un groupement $(CH_2)_nCN$
- un groupement $(CH_2)_nCHO$
- un groupement $(CH_2)_{(n+1)}OH$

dans lequel n est un nombre entier compris entre 1 et 6,
$R_1$ et $R_2$ étant définis dans la description, et leur utilisation comme médicaments.

# NOUVEAUX DERIVES MACROLIDES, LEUR PROCEDE DE PREPARATION ET LES COMPOSITIONS PHARMACEUTIQUES QUI LES CONTIENNENT

La présente invention concerne de nouveaux antibiotiques de la famille des macrolides, leur procédé de préparation et les compositions pharmaceutiques qui les contiennent.

Les besoins de la thérapeutique exigent le développement constant de nouveaux antibiotiques à la fois en raison de la possibilité d'apparition de nouvelles souches résistantes, mais également dans le but de créer de nouvelles molécules possédant une activité améliorée aussi bien au niveau de leur seuil d'efficacité que de l'étendue de leur spectre d'action.

De nombreuses modifications du noyau de la tylosine ont déjà été réalisées afin de produire de nouveaux antibiotiques intéressants. Parmi les plus récentes, citons les brevets US 4.528 369, 4 581 346, 4 629 786 et les demandes de brevets européens 0 103 465, 0 104 028, 0 154 495 et 0 203 621 et 0 292 852. Toutefois, aucune de ces modifications n'a permis d'obtenir un dérivé de la tylosine utilisé en thérapeutique humaine.

Plus particulièrement, la présente invention a pour objet les produits dérivés de la tylosine de formule générale (I) :

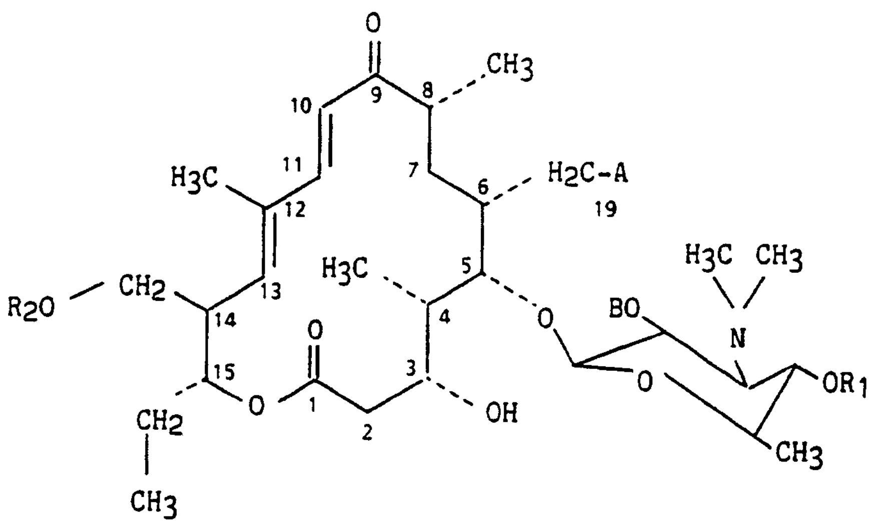

(I)

dans laquelle :

- $R_1$ représente un atome d'hydrogène ou un groupement :

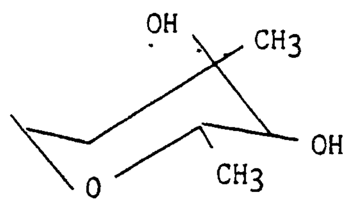

- $R_2$ représente un atome d'hydrogène ou un groupement :

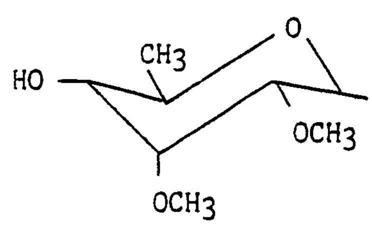

et dans le cas où $R_2$ représente un atome d'hydrogène, $R_1$ représente également un atome d'hydrogène,

- B représente un atome d'hydrogène ou un groupement acyle inférieur,
- A représente :
- un groupement $CH_2OH$ et dans ce cas B représente un groupement acyle inférieur, ou bien :
- un groupement $(CH_2)_nCN$,
- ou un groupement - $(CH_2)_nCHO$,
- ou un groupement - $(CH_2)_{(n+1)}OH$,

dans lequel n est un nombre entier compris entre 1 et 6, étant entendu que par groupement acyle inférieur, on entend un groupement comprenant de 1 à 5 atomes de carbone.

L'invention s'étend également aux sels des composés de formule (I). Parmi les acides que l'on peut ajouter aux composés de formule (I) pour former un sel d'addition, on peut citer, à titre d'exemple, les acides chlorhydrique, bromhydrique, iodhydrique, sulfurique, acétique, propionique, trifluoroacétique, maléique, malique, tartrique, méthanesulfonique, éthanesulfonique, benzènesulfonique, p-toluènesulfonique, phosphorique, fumarique, citrique, camphorique, stéarique, éthyl succinique...

La présente invention s'étend également au procédé de préparation des dérivés de formule (I) caractérisé en ce que l'on utilise comme matière première un dérivé de formule (II) :

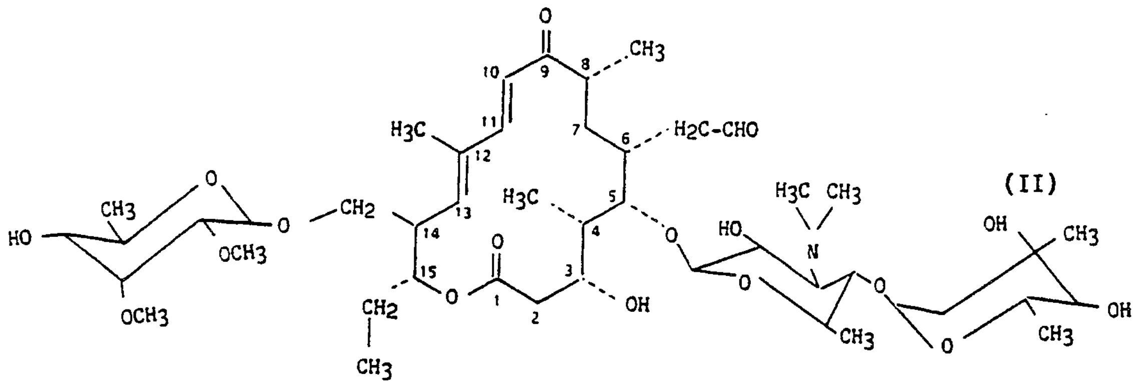

(II)

. que l'on soumet, lorsque dans le dérivé de formule (I) que l'on souhaite obtenir $R_1$ représente un atome d'hydrogène, à l'action de l'acide chlorhydrique dilué de normalité comprise entre 0,05 et 0,4 préférentiellement comprise entre 0,1 et 0,30 préférentiellement comprise entre 0,15 et 0,25, à température ambiante, pour conduire, après lavage par un solvant organique approprié, alcalinisation et extraction par un solvant organique approprié à un dérivé de formule (II/A) :

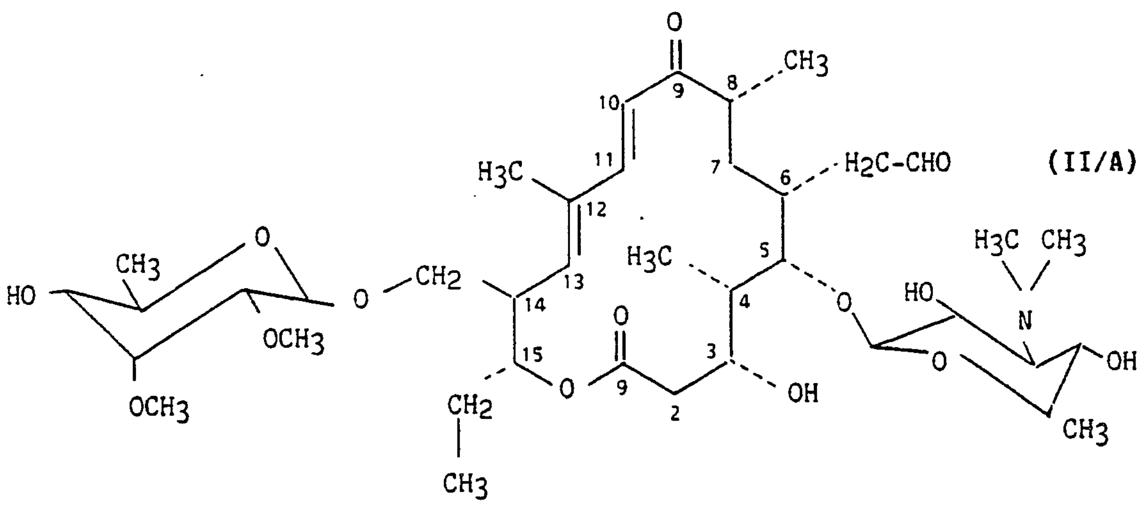

(II/A)

dérivé de formule (II/A) que l'on fait réagir, lorsque dans le dérivé de formule (I) que l'on souhaite obtenir $R_2$ représente un atome d'hydrogène, avec l'acide chlorhydrique de normalité comprise entre 0,25 et 0,75, préférentiellement entre 0,3 et 0,7, préférentiellement entre 0,4 et 0,6, à une température comprise préférentiellement entre 30 et 100 °C ; préférentiellement comprise entre 50 et 90 °C, préférentiellement entre 70 et 80 °C, pour conduire, après lavage par un solvant organique approprié, alcalinisation et extraction par un solvant organique approprié à un dérivé de formule (II/B) :

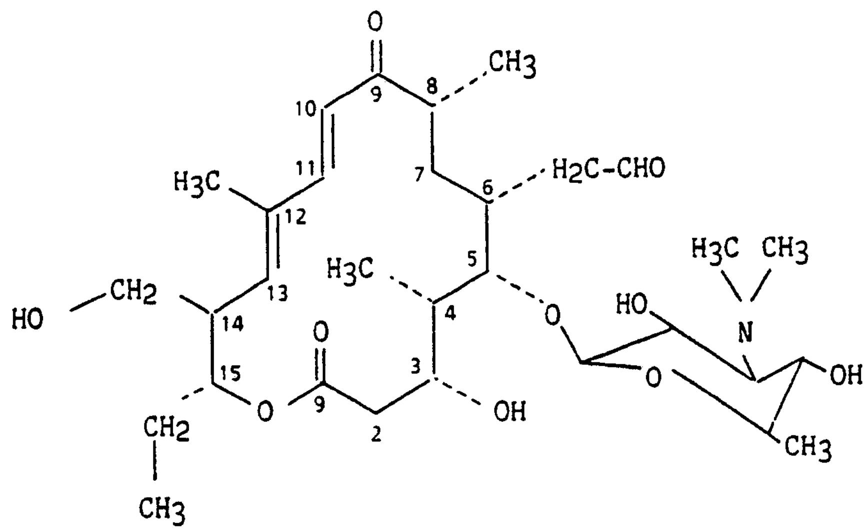

(II/B)

dérivé de formule (II), (II/A) ou (II/B), selon le dérivé de formule (I) que l'on souhaite obtenir, qui est soumis ou bien :

1/ à l'action d'un hydrure mixte de métal alcalin en milieu d'alcool aliphatique inférieur pour obtenir un dérivé de formule (III) :

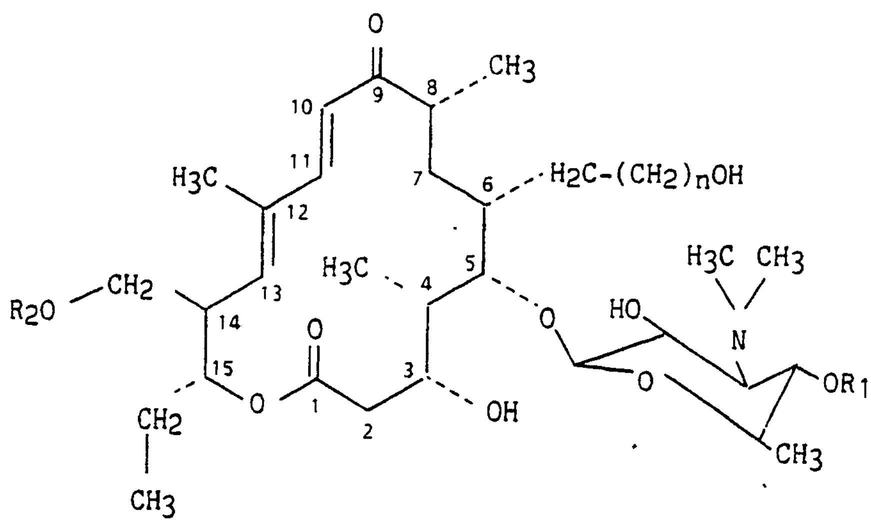

(III)

dans laquelle $R_1$ et $R_2$ ont la même signification que dans la formule (I), et n représente 1,

que l'on soumet à l'action d'un anhydride d'acide carboxylique inférieur de formule Ac-OH dans laquelle Ac représente un groupement acyle inférieur, à température préférentiellement ambiante et préférentiellement sous atmosphère inerte pour obtenir un dérivé de formule (IV) :

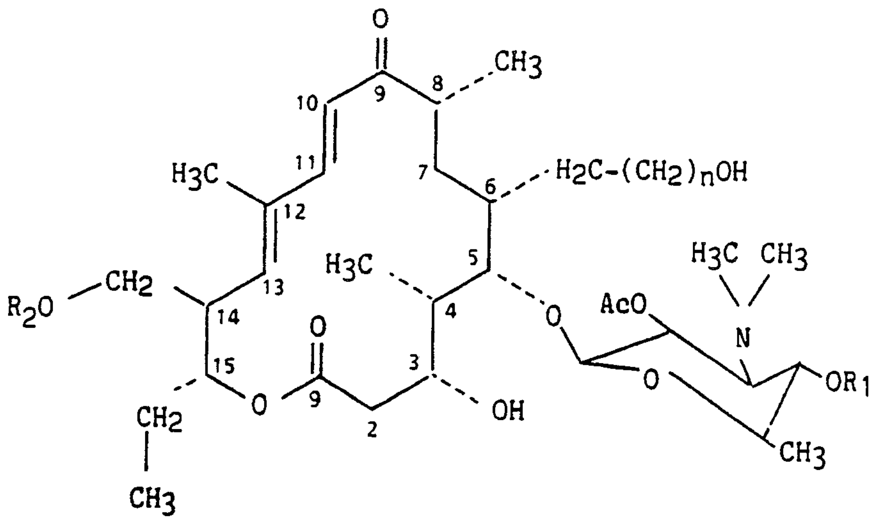

(IV)

dans laquelle Ac représente un groupement acyle inférieur, n vaut 1, $R_1$ et $R_2$ ont la même définition que dans la formule (I),
cas particulier de dérivés de formule (I) pour lesquels B représente un groupement acyle inférieur et A représente un groupement $CH_2OH$,
que l'on traite par le chlorure de tosyle, que l'on ajoute par petites fractions,
en présence de diméthylamino-4 pyridine pour obtenir après éventuelle purification, extraction et éventuelle chromatographie, un dérivé de formule (V) :

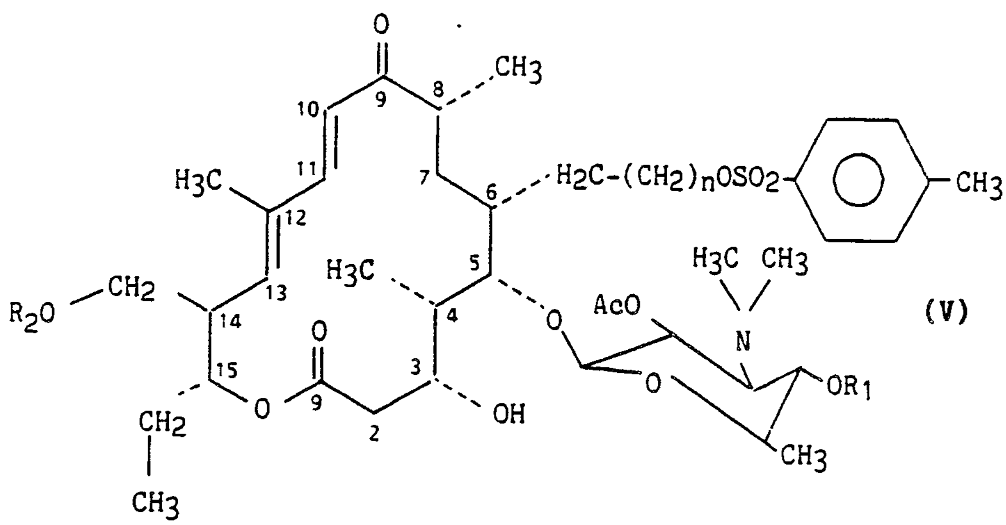

(V)

dans laquelle n, Ac, $R_1$ et $R_2$ ont la même signification que dans la formule (IV),
que l'on traite par un cyanure de métal alcalin en présence d'un agent cryptant pour obtenir un dérivé de formule (VI) :

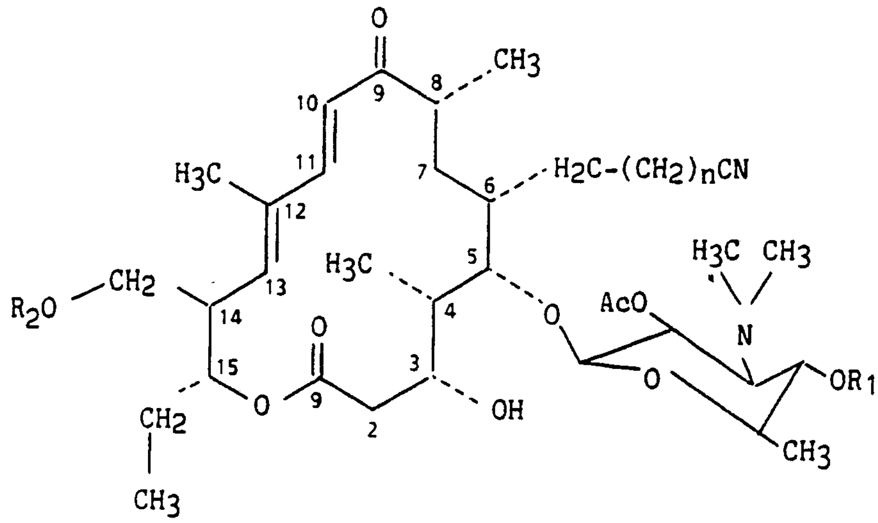

(VI)

dans laquelle n, Ac, $R_1$ et $R_2$ ont la même signification que dans la formule (IV), cas particulier de dérivés de formule (I) pour lesquels B représente un groupement acyle inférieur et A représente un groupement $CH_2CN$,
que l'on soumet à action du méthanol,
pour obtenir un dérivé de formule (VII) :

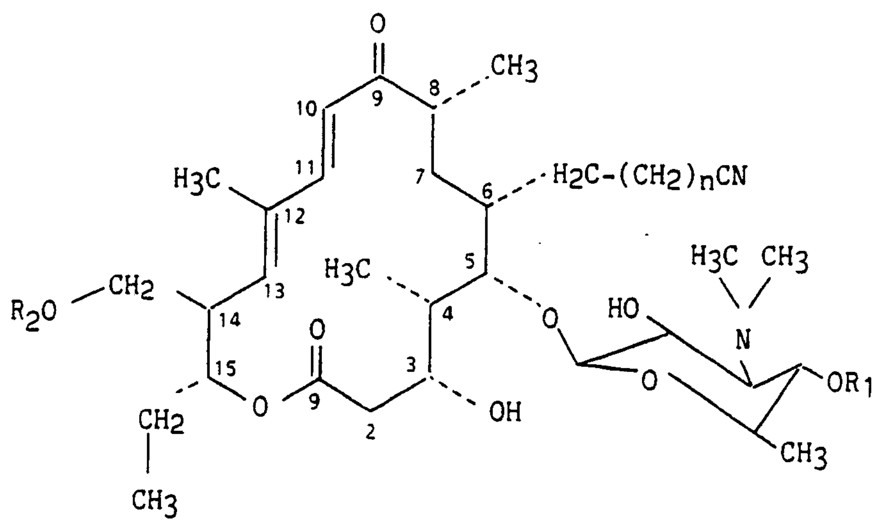

(VII)

dans laquelle $R_1$ et $R_2$ ont la même définition que dans la formule (I), et n représente 1,
cas particulier des dérivés de formule (I) pour lesquels B représente un atome d'hydrogène et A représente un groupement $CH_2CN$,
que l'on traite par le diisobutyl hydrure d'aluminium préférentiellement sous atmosphère inerte, pour conduire à un dérivé de formule (VIII) :

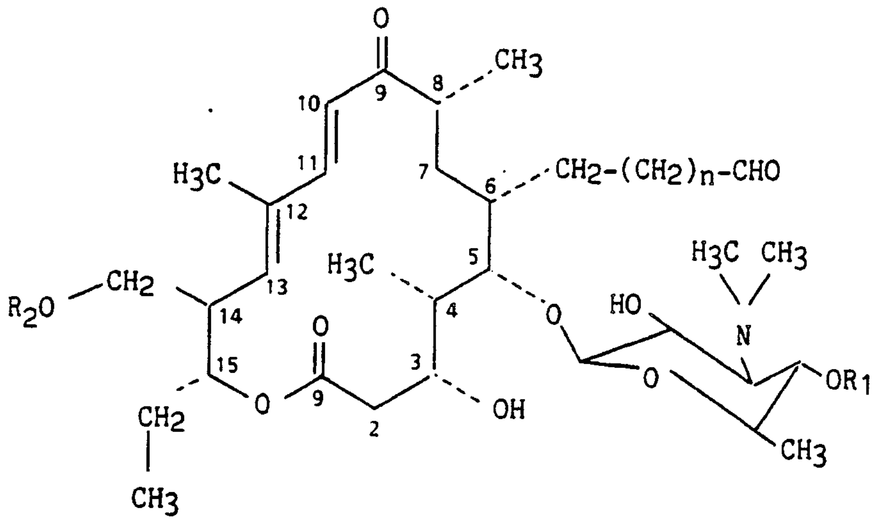

(VIII)

dans laquelle $R_1$ et $R_2$ ont la même définition que dans la formule (I), et n représente 1,
cas particulier des dérivés de formule (I) pour lesquels B représente un atome d'hydrogène et A représente un groupement $CH_2$-CHO,
2/ ou bien à l'action du chlorure de (paratoluène sulfonyle) méthyle en présence du n tert.butylate de métal alcalin dans un alcool aliphatique inférieur, pour conduire après éventuelle purification par chromatographie à un dérivé de formule (IX) :

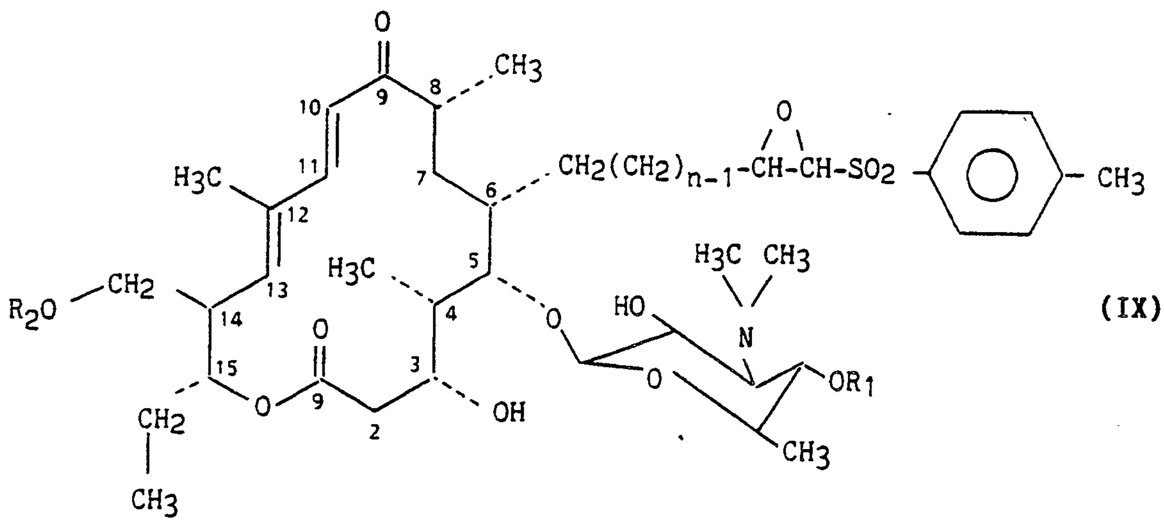

(IX)

dans laquelle $R_1$ et $R_2$ ont la même définition que dans la formule (I), et n représente 1,
que l'on traite par un hydrure mixte de métal alcalin en milieu de diméthylformamide préférentiellement sous léger chauffage, pour conduire après éventuelle purification par chromatographie à un dérivé de formule (X) :

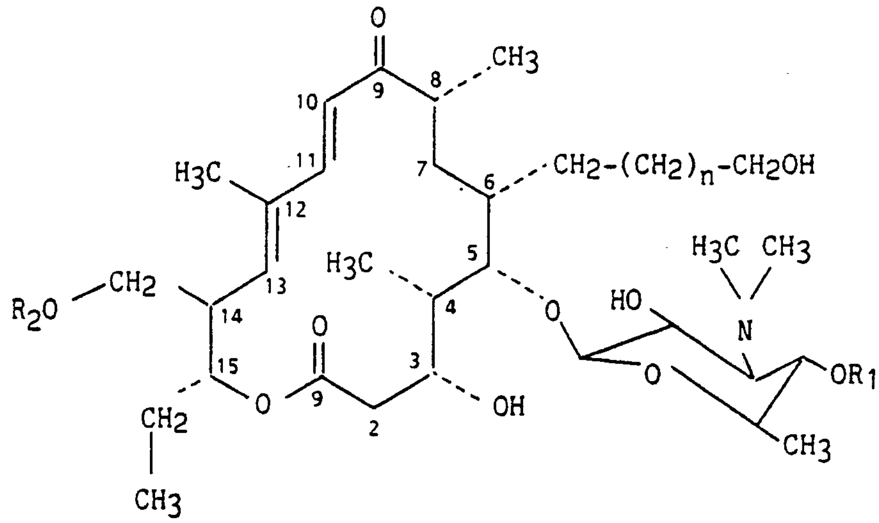

(X)

dans laquelle $R_1$ et $R_2$ ont la même définition que dans la formule (I), et n représente 1,
cas particulier des dérivés de formule (I) pour lesquels A représente un groupement $(CH_2)_2$-OH et B représente un atome d'hydrogène,
que l'on traite par le chlorochromate de pyridinium pour obtenir, après éventuelle purification par chromatographie, un produit de formule (VIII),
lequel à son tour, si on le désire peut être soumis, comme indiqué plus haut, en fonction du dérivé de formule (I) que l'on souhaite obtenir,
- soit 3/ à l'action successive d'un hydrure mixte de métal alcalin d'anhydride d'acide carboxylique inférieur, de chlorure de tosyle en présence de diméthyl amino - 4 pyridine, de cyanure de métal alcalin en présence d'un agent cryptant, de méthanol puis de diisobutyl hydrure d'aluminium, pour obtenir successivement les dérivés de formule (III/A), (IV/A), (V/A), (VI/A), (VII/A) et (VIII/A) cas particulier des dérivés de formule (III), (IV), (V), (VI), (VII) et (VIII), pour lesquels n représente 2, $R_1$ et $R_2$ ayant la même signification que dans la formule (I), Ac signifiant le cas échéant un groupement acyle inférieur,
- soit 4/ à l'action successive de chlorure de (paratoluène sulfonyle) méthyle en présence de tert.butylate de métal alcalin, puis d'hydrure mixte de métal alcalin en présence de diméthylformamide puis de chlorochromate de pyridinium, pour conduire successivement aux dérivés de formule (IX/A), (X/A) et (VIII/A) cas particulier des dérivés de formules (IX), (X) et (VIII), pour lesquels n représente 2, $R_1$ et $R_2$ ayant la même signification que dans la formule (I),
dérivé de formule (VIII/A) qui à son tour est éventuellement soumis à une ou deux, trois ou quatre reprises à un traitement identique à ceux indiqués en 3/ ou 4/, pour conduire à un dérivé de formules (III/B), (IV/B), (V/B), (VI/B), (VII/B), (VIII/B), (IX/B), (X/B) cas particulier des dérivés de formules (III), (IV), (V), (VI), (VII), (VIII), (IX), (X) pour lesquels n représente 3, 4, 5 ou 6, selon que le traitement indiqué en 3/ ou 4/ est exécuté à 1, 2, 3 ou 4 reprises, R1 et R2 ayant la même signification que dans la formule (I) et Ac signifiant le cas échéant un groupement acyle inférieur,
dérivés que l'on purifie éventuellement par chromatographie sur colonne de silice à l'aide d'un mélange solvant approprié,
et que l'on peut, si on le désire salifier par un acide pharmaceutiquement acceptable.

Les composés de formule (V) et les composés de formule (IX) et plus généralement les composés de formule (I) pour lequel A représente un groupement - $(CH_2)nOSO_2$ - $C_6H_4$ - $(p)CH_3$ et B représente un groupement acétyle,
ou A représente

$$-(CH_2)_{n-1} - \overset{\frown O \frown}{CH - CH} - SO_2 - C_6H_4 - (p)CH_3,$$

n étant compris entre 1 et 6, sont nouveaux et font partie de l'invention au même titre que les dérivés de formule (I) dont ils constituent les intermédiaires de synthèse.

Les composés de formule (I) possèdent des propriétés pharmacologiques intéressantes.

En particulier, ces composés sont actifs sur le cocci gram + et cocci gram -, les bacilles gram +

(clostridies), certains bacilles gram -, hémophilus (ex : hémophilus influenzae), nesseiria gonorrhoeae, brucella, bordetella, les anaérobies, les mycoplasmes, les rickettsies et les myagawanelles (chlamidia), les spirochètes, les protozoaires et certains dermofongi.

Plus particulièrement, les composés de formule (I) possèdent une très bonne activité antibiotique sur les pneumocoques, les staphylocoques et les streptocoques. Ce spectre d'activité rend les composés de formule (I) particulièrement intéressants dans le traitement d'un grand nombre d'affections ; parmi celles-ci, il est possible de citer, à titre d'exemple, les pneumococcies telles que les bronchites, la brucellose, la diphtérie, la gonococcie, les pneumonies, les streptococcies telles que les angines aiguës, les otites, la scarlatine, les sinusites, les staphylococcies telles que septicémies à staphylocoques, anthrax, érésipèles, pyodermites, staphylococcies aiguës, broncho pneumonies et suppurations pulmonaires.

De plus, les composés de la présente invention, de par leur structure sont susceptibles de se révéler intéessants en raison de leur absence de toxicité hépatique ou gastrointestinale, ce qui les distingue avantageusement d'autres familles de composés antibiotiques.

La présente invention a également pour objet les compositions pharmaceutiques contenant les produits de formule (I) où un de leurs sels d'addition à un acide pharmaceutiquement acceptable, seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, perlinguale, oculaire ou respiratoire, et notamment les préparations injectables, les aérosols, les gouttes oculaires ou nasales, les comprimés simples ou dragéifiés, les comprimés sublinguaux, les sachets, les paquets, les gélules, les glossettes, les tablettes, les suppositoires, les crèmes, pommades, gels dermiques, etc...

Les compositions pharmaceutiques selon l'invention peuvent également se présenter sous forme d'une poudre lyophilisée qui est destinée à être dissoute au moment de l'emploi dans un solvant approprié notamment l'eau stérile apyrogène.

La posologie varie selon l'âge et le poids du patient, la voie d'administration, la nature de l'indication thérapeutique et des traitements éventuellement associés et s'échelonne entre 1 centigramme et 4 grammes par prise ou par application.

Les exemples suivants illustrent l'invention et ne la limitent en aucune façon.

Les spectres de résonance magnétique nucléaire du $^{13}C$ et du $^{1}H$ ont été enregistrés en utilisant le TMS comme référence interne.

La matière première utilisée dans la synthèse des composés de formule (I) est la tylosine connue dans la littérature.

**EXEMPLE 1 : ACETYL - 2′ DIHYDRO - 20 TYLOSINE**

**STADE A : DIHYDRO - 20 TYLOSINE**

Dissoudre 0,916 g (0,01 mole) de tylosine dans le méthanol. Ajouter par petites portions sous agitation et à température ambiante 0,037 g (0,014 mole) de borohydrure de sodium en une heure environ. Maintenir l'agitation pendant trois heures à température ambiante. Evaporer à sec, reprendre le résidu par l'eau et extraire à 3 reprises au chloroforme. Sécher sur chlorure de calcium. Filtrer et évaporer le chloroforme au bain-marie sous vide.

**STADE B : ACETYL - 2′ DIHYDRO - 20 TYLOSINE**

Dissoudre 0,29 g (0,3 x $10^{-3}$ mole) de dihydro - 20 tylosine obtenue au stade A dans 20 ml d'acétone anhydre. Additionner 0,001 mole d'anhydride acétique et agiter une nuit à température ambiante sous atmosphère d'azote. Evaporer. Reprendre le résidu par de l'eau saturée en chlorure de sodium et extraire au chlorure de méthylène. Reprendre la phase organique, sécher sur sulfate de sodium et évaporer au bain-marie sous vide. Purifier par passage en chromatographie sur plaque (mélange éluant : chlorure de méthylène, méthanol, ammoniaque 20/1/0,05).

Rendement : 50 %

Caractéristiques spectrales :

Résonance magnétique nucléaire $^{13}C$ ($\delta$ ppm) :

204 : $C_9$
174 : $C_1$
169 : (C=0) acétate
Masse : FAB $[M-H]^+$ : 961

**EXEMPLE 2 : CYANOMETHYL - 19 DEFORMYL - 19 ACETYL - 2′ TYLOSINE**

**STADE A : O - PARATOLUENESULFONYL - 20 HYDRO - 20 O ACETYL - 2′ TYLOSINE**

Dissoudre 0,106 g (1,1 x $10^{-4}$ mole) de dihydro - 20 acétyl - 2′ tylosine obtenue dans l'exemple 1 en présence de traces de diméthylamino -4 pyridine dans 3 ml de dichlorométhane et 0,3 ml de pyridine. Ajouter 0,03 g (1,57 x $10^{-4}$ mole) de chlorure de tosyle à température ambiante sous argon. Après trois heures d'agitation, ajouter 0,018 g (1,1 x $10^{-4}$ mole) de chlorure de tosyle. Après 17 heures d'agitation, ajouter à nouveau 2 mg de chlorure de tosyle. Après 22 heures d'agitation, ajouter 0,2 ml de méthanol. Extraire ensuite par un mélange eau-bicarbonate de sodium/chlorure de méthylène (50/50), sécher sur sulfate de sodium et purifier par flash chromatographie (éluant : chlorure de méthylène, méthanol, ammoniaque/20/1/0,05).
Rendement : 60 %
Caractéristiques spectrales :
Spectrométrie de masse : FAB $[M-H]^+$ : 1115
Résonance magnétique nucléaire :
RMN $^1H$
$\delta$ = 1,75 ppm, singulet 3H $C_6H_4$-$CH_3$
$\delta$ = 7,05-7,28 ppm 4H aromatiques
RMN $^{13}C$
$\delta$ = 203 ppm $C_9$
$\delta$ = 174 ppm C1
$\delta$ = 169 ppm (COO) acétate
$\delta$ = 29,3 ppm $CH_3$-$C_6H_4$

**STADE B : CYANOMETHYL - 19 DEFORMYL - 19 ACETYL - 2′ TYLOSINE**

Dissoudre 0,035 g (0,53 x $10^{-3}$ mole) de cyanure de potassium et 0,217 g (0,57 x $10^{-3}$ mole) d'éther couronne (18 crown - 6 - dicyclohexyle) par sonication dans 5 ml d'acétonitrile anhydre sous argon. Ajouter ensuite goutte à goutte et sous agitation une solution de 0,5 g (0,45 x $10^{-3}$ mole) d'O paratoluène sulfonyl - 20 hydro - 20 O acétyl - 2′ tylosine obtenue au stade A dans 4 ml d'acétonitrile. Maintenir l'agitation durant une nuit à température ambiante. Evaporer le milieu réactionnel et purifier le résidu sur colonne de silice (éluant : $CH_2Cl_2$/MeOH/$NH_4OH$-30/1/0,05)
$\alpha$ D : -62,4°
Caractéristiques spectrales :
Spectrométrie de masse : FAB $[M-H]^+$ : 970
Résonance magnétique nucléaire $^{13}C$ ($\delta$ ppm) :
RMN $^1H$
$\delta$ = 2,1 ppm, singulet 3H $C_0$-$CH_3$
RMN $^{13}C$
$\delta$ = 203 ppm, $C_9$

**EXEMPLE 3 : CYANOMETHYL - 19 DEFORMYL - 19 TYLOSINE**

Dissoudre 0,97 g (1 millimole) de cyanométhyl - 19 déformyl - 19 acétyl - 2′ tylosine obtenue dans l'exemple 2 dans 15 ml de méthanol. Agiter 4 heures à température ambiante. Evaporer le milieu réactionnel et extraire le résidu à 2 reprises par 10 ml de chloroforme. Réunir les phases organiques. Sécher sur sulfate de sodium, filtrer et évaporer.
Rendement : 90 %

Caractéristiques spectrales :
Spectrométrie de masse : FAB $[M-H]^+$ : 928
Résonance magnétique nucléaire $^{13}C$ (δ ppm) :
RMN $^{13}C$
δ = 203 ppm, $C_9$

**EXEMPLE 4 : FORMYLMETHYL - 19 DEFORMYL - 19 TYLOSINE**

Dissoudre sous argon dans un mélange chlorure de méthylène/toluène anhydre (1/1 v/v) 0,93 g (1 millimole) de cyano méthyl - 19 déformyl - 19 tylosine obtenue dans l'exemple 3. Ajouter lentement, en maintenant la température à -20° C, 2 millimole d'hydrure de diisobutyl aluminium. Laisser revenir la température à l'ambiante et maintenir l'agitation pendant 3 heures. Ajouter une solution saturée de chlorure d'ammonium et extraire à trois reprises par 10 ml de chlorure de méthylène. Sécher sur sulfate de sodium. Filtrer et évaporer le milieu réactionnel. Purifier par flash chromatographie (éluant : méthanol/chlorure de méthylène/ammoniaque 9,5/90/0,5).
Rendement : 70 %
Caractéristiques spectrales :
Spectrométrie de masse : FAB $[M-H]^+$ : 925
Résonance magnétique nucléaire :
RMN $^{13}C$
δ = 203 ppm, $C_9$

**EXEMPLE 5 : DEFORMYL - 19 (HYDROXY - 2 ETHYL) - 19 TYLOSINE**

**STADE A : (PARATOLUENE SULFONYL - 3 EPOXY - 2,3) PROPYL - 6 DEFORMYLMETHYL - 6 TYLOSINE**

Dissoudre 3,36 g (4 mmole) de tylosine et 820 mg (4 mmole) de chlorure de (paratolylsulfonyl)méthyle dans 32 ml de tétrahydrofuranne anhydre et 2,3 ml de tert.butanol sous azote. Refroidir par un bain de glace. Ajouter 448 mg (4 mmole) de tert.butylate de potassium. Agiter le mélange réactionnel à température ambiante pendant 2 heures. Ajouter de l'éther, filtrer et évaporer à sec. Le produit brut est purifié par flash chromatographie sur colonne de silice (éluant : chlorure de méthylène/méthanol/ammoniaque (20/1/0,05).
Rendement : 70 %
Caractéristiques spectrales :
Spectrométrie de masse : FAB $[M-H]^+$ : 1085
Résonance magnétique nucléaire [1H] :
δ = 2,5 ppm, singulet, 3H, $C_6H_5$-$CH_3$

**STADE B : DEFORMYL - 19 (HYDROXY - 2 ETHYL) - 19 TYLOSINE**

Dissoudre 2,4 g de (paratoluène sulfonyl - 3 époxy - 2,3) propyl - 6 deformylméthyl - 6 tylosine obtenue au stade précédent dans 40 ml de diméthyl formamide. Ajouter 60 mg de borohydrure de sodium. Agiter le mélange réactionnel à une température de 80° C pendant 90 minutes. Evaporer à sec le milieu réactionnel et purifier le résidu par flash chromatographie (éluant : chlorure de méthylène/méthanol ammoniaque/20/1/0,05).
Rendement : 75 %
Caractéristiques spectrales :
Résonance magnétique nucléaire :
RMN $^{13}C$
δ = 203 ppm, $C_9$
δ = 174 ppm, $C_1$
δ = 129 ppm, $CH_2O_4$

**EXEMPLE 6 : DEFORMYL - 19 FORMYLMETHYL - 19 TYLOSINE**

A une solution de 100 mg de tamis moléculaire 3Å dans le chlorure de méthylène, ajouter 35 mg de chlorochromate de pyridinium. Après 3 minutes ajouter 100 mg de déformyl - 19 (hydroxy - 2 éthyl) - 19 tylosine obtenue dans l'exemple 5. Agiter le mélange réactionnel à température ambiante pendant 2 heures. Ajouter de l'éther éthylique et filtrer. Evaporer à sec et purifier sur plaque préparative (éluant : chlorure de méthylène/méthanol/ammoniaque).
Spectrométrie de masse : FAB $[M-H]^+$ : 931
Caractéristiques spectrales :
Résonance magnétique nucléaire :
RMN $^{13}C$
$\delta$ = 203,5 ppm, $C_9$
$\delta$ = 202,8 ppm, $C_{21}$
$\delta$ = 174 ppm, $C_1$

**EXEMPLE 7 : DEFORMYL - 19 (HYDROXY - 2 ETHYL) - 19 DEMYCAROSYL TYLOSINE**

**STADE A : DEMYCAROSYL TYLOSINE**

Agiter 4 g (0,004 mmole) de tylosine base dans 80 ml d'acide chlorhydrique 0,2 N pendant 4 heures à la température ambiante. Laver le milieu réactionnel obtenu au dichlorométhane, séparer la phase aqueuse et l'ajuster à PH 8,0. Extraire à deux reprises par 120 ml de dichlorométhane, réunir les phases organiques, sécher sur sulfate de sodium et évaporer. Le résidu est constitué de démycarosyl tylosine.
Rendement : 94 %

**STADE B : DEFORMYL - 19 (HYDROXY - 2 ETHYL) - 19 DEMYCAROSYL TYLOSINE**

En procédant comme dans l'exemple 5, mais en remplaçant au stade A, la tylosine par la démycarosyl tylosine, on obtient le produit du titre.
Rendement : 65 %
Caractéristiques spectrales :
Spectrométrie de masse : FAB $[M-H]^+$ : 789

**EXEMPLE 8 : DEFORMYL - 19 FORMYLMETHYL - 19 DEMYCAROSYL TYLOSINE**

En procédant comme dans l'exemple 6, mais en remplaçant la déformyl - 19 (hydroxy - 2 éthyl) - 19 tylosine par la déformyl - 19 (hydroxy - 2 éthyl)- 19 démycarosyl tylosine, on obtient le produit du titre.
Caractéristiques spectrales :
Spectrométrie de masse : FAB $[M-H]^+$ : 787
Résonance magnétique nucléaire :
RMN $^{13}C$
$\delta$ = 204 ppm, $C_9$
$\delta$ = 174 ppm, $C_1$
$\delta$ = 203 ppm, $C_{21}$

**EXEMPLE 9 : DEFORMYL - 19 (HYDROXY - 2 ETHYL) - 19 DEMYCAROSYL DEMYCINOSYL TYLOSINE**

**STADE A : DEMYCAROSYL DEMYCINOSYL TYLOSINE**

Dissoudre 5 g (0,0065 mmole) de démycarosyl tylosine obtenue au stade A de l'exemple 7, dans 110 ml d'acide chlorhydrique 0,5 N et agiter pendant 27 heures environ à 75 °C. Laver le milieu réactionnel au

dichlorométhane. Récupérer la phase aqueuse, l'ajuster à PH 8 et extraire à deux reprises par 120 ml de dichlorométhane. Sécher la phase organique sur sulfate de sodium et évaporer. Le résidu est chromatographié sur gel de silice (éluant : $CH_2Cl_2$/MEOH/$NH_4OH$/10/1/0,05) pour obtenir le produit attendu.
Rendement : 20 %

**STADE B : DEFORMYL - 19 (HYDROXY - 2 ETHYL) - 19 DEMYCAROSYL DEMYCINOSYL TYLOSINE**

En procédant comme dans l'exemple 5, mais en remplaçant la tylosine par la démycarosyl démycinosyl tylosine, on obtient le produit du titre.
Rendement : 50 %
Caractéristiques spectrales :
Spectrométrie de masse : FAB $[M-H]^+$ : 614

**EXEMPLE 10 : DEFORMYL - 19 FORMYLMETHYL - 19 DEMYCAROSYL DEMYCINOSYL TYLOSINE**

En procédant comme dans l'exemple 6, mais en remplaçant la déformyl - 19 (hydroxy - 2 éthyl) - 19 tylosine par la déformyl - 19 (hydroxy - 2 éthyl) - 19 démycarosyl démycinosyl tylosine obtenue dans l'exemple 9, on obtient le produit du titre.
Caractéristiques spectrales :
Spectrométrie de masse : FAB $[M-H]^+$ : 612
Résonance magnétique nucléaire :
RMN $^{13}C$
$\delta$ = 204 ppm, $C_9$
$\delta$ = 174 ppm, $C_1$
$\delta$ = 203 ppm, $C_{21}$

**EXEMPLE 11 : DEFORMYL - 19 (HYDROXY - 3 PROPYL) - 19 TYLOSINE**

En procédant comme dans l'exemple 5, mais en remplaçant la tylosine par la déformyl - 19 formyl méthyl - 19 tylosine obtenue dans l'exemple 6, on obtient le produit du titre.
Caractéristiques spectrales :
Spectrométrie de masse : FAB $[M-H]^+$ : 947

**EXEMPLE 12 : DEFORMYL - 19 (FORMYL - 2 ETHYL) - 19 TYLOSINE**

En procédant comme dans l'exemple 6, mais en remplaçant la déformyl - 19 (hydroxy - 2 éthyl) - 19 tylosine par la déformyl - 19 (hydroxy - 3 propyl) - 19 tylosine obtenue dans l'exemple précédent, on obtient le produit du titre.
Caractéristiques spectrales :
Spectrométrie de masse : FAB $[M-H]^+$ : 945
Résonance magnétique nucléaire :
RMN $^{13}C$
$\delta$ = 203 ppm, $C_9$
$\delta$ = 174 ppm, $C_1$

**EXEMPLE 13 : DEFORMYL - 19 (HYDROXY - 4 n BUTYL) - 19 TYLOSINE**

En procédant comme dans l'exemple 5, mais en remplaçant la tylosine par la déformyl - 19 (formyl - 2 éthyl) - 19 tylosine obtenue dans l'exemple 12, on obtient le produit du titre.
Caractéristiques spectrales :
Spectrométrie de masse : FAB $[M-H]^+$ : 961

**EXEMPLE 14 : DEFORMYL - 19 (FORMYL - 3 n PROPYL) - 19 TYLOSINE**

En procédant comme dans l'exemple 6, mais en remplaçant la déformyl - 19 (hydroxy - 2 éthyl) - 19 tylosine par la déformyl - 19 (hydroxy - 4 n butyl)- 19 tylosine obtenue dans l'exemple précédent, on obtient le produit du titre.
Caractéristiques spectrales :
Spectrométrie de masse : FAB $[M-H]^+$ : 959
Résonance magnétique nucléaire :
RMN $^{13}C$
$\delta$ = 203 ppm, $C_9$
$\delta$ = 174 ppm, $C_1$

**EXEMPLE 15 : DEFORMYL - 19 (HYDROXY - 5 n PENTYL) - 19 TYLOSINE**

En procédant comme dans l'exemple 5, mais en remplaçant la tylosine par la déformyl - 19 (formyl - 3 n propyl) - 19 tylosine, on obtient le produit du titre.
Caractéristiques spectrales :
Spectrométrie de masse : FAB $[M-H]^+$ : 975

**EXEMPLE 16 : DEFORMYL - 19 (FORMYL - 4 n BUTYL) - 19 TYLOSINE**

En procédant comme dans l'exemple 6, mais en remplaçant la déformyl - 19 (hydroxy - 2 éthyl) - 19 tylosine par la déformyl - 19 (hydroxy - 5 n pentyl) - 19 tylosine, on obtient le produit du titre.

**EXEMPLE 17 : ACETYL - 2′ DEFORMYL - 19 (HYDROXY - 2 ETHYL) - 19 TYLOSINE**

En procédant comme dans l'exemple 1, mais en remplaçant la tylosine, au stade A, par la formylméthyl - 19 déformyl - 19 tylosine obtenue dans l'exemple 4 ou 6, on obtient le produit du titre.
Caractéristiques spectrales :
Spectrométrie de masse : FAB $[M-H]^+$ : 975
Résonance magnétique nucléaire :
RMN $^{13}C$
$\delta$ = 204 ppm, $C_9$
$\delta$ = 174 ppm, $C_1$
$\delta$ = 169 (C=O-O) acétate

**EXEMPLE 18 : (CYANO - 2 ETHYL) - 19 DEFORMYL - 19 ACETYL - 2′ TYLOSINE**

En procédant comme dans l'exemple 2, mais en remplaçant la dihydro -20 acétyl - 2′ tylosine par l'acétyl - 2′ déformyl - 19 (hydroxy - 2 éthyl) - 19 tylosine obtenue dans l'exemple 17, on obtient le produit du titre.
Caractéristiques spectrales :
Spectrométrie de masse : FAB $[M-H]^+$ : 984

**EXEMPLE 19 : (CYANO - 2 ETHYL) - 19 DEFORMYL - 19 TYLOSINE**

En procédant comme dans l'exemple 3, mais en remplaçant la cyanométhyl - 19 déformyl - 19 acétyl - 2′ tylosine par la (cyano - 2 éthyl) - 19 déformyl - 19 acétyl - 2′ tylosine obtenue dans l'exemple 18, on obtient le produit du titre.
Caractéristiques spectrales :
Spectrométrie de masse : FAB $[M-H]^+$ : 942

**EXEMPLE 20 : ACETYL - 2' DEFORMYL - 19 (HYDROXY - 3 n PROPYL) - 19 TYLOSINE**

En procédant comme dans l'exemple 1, mais en remplaçant la tylosine au stade A par la (formyl - 2 éthyl) - 19 déformyl - 19 tylosine obtenue dans l'exemple 12, on obtient le produit du titre.

Caractéristiques spectrales :
Spectrométrie de masse : FAB $[M-H]^+$ : 989
Résonance magnétique nucléaire :
RMN $^{13}C$
$\delta$ = 204 ppm, $C_9$
$\delta$ = 174 ppm, $C_1$

**EXEMPLE 21 : (CYANO - 3 n PROPYL) - 19 DEFORMYL - 19 ACETYL - 2' TYLOSINE**

En procédant comme dans l'exemple 2, mais en remplaçant la dihydro -acétyl - 2' tylosine par l'acétyl - 2' déformyl - 19 (hydroxy - 3 n propyl) - 19 tylosine obtenue dans l'exemple 20, on obtient le produit du titre.

Caractéristiques spectrales :
Spectrométrie de masse : FAB $[M-H]^+$ : 1129

**EXEMPLE 22 : (CYANO - 3 n PROPYL) - 19 DEFORMYL - 19 TYLOSINE**

En procédant comme dans l'exemple 3, mais en remplaçant la cyanométhyl - 19 déformyl - 19 acétyl - 2' tylosine par la (cyano - 3 n propyl) - 19 déformyl - 19 acétyl - 2' tylosine obtenue dans l'exemple 21, on obtient le produit du titre.

Caractéristiques spectrales :
Spectrométrie de masse : FAB $[M-H]^+$ : 956

**EXEMPLE 23 : ETUDE DE L'ACTIVITE DES PRODUITS DE FORMULE (I) SUR DIVERSES SOUCHES BACTERIENNES**

La détermination des concentrations minimales inhibitrices (CMI) est effectuée :

- pour les Staphylocoques et les Entérocoques (Streptocoques D) en milieu gélosé ou liquide de MUELLER HINTON ;
- pour les Hémophilus, Streptocoques non D et Neisseria Gonorrhoeae, la détermination des CMI est effectuée suivant la méthode de dilution en milieu gélosé au sang cuit enrichi du mélange Polyvitex*. La culture est faite en atmosphère enrichie en $CO_2$.

La lecture des CMI est effectuée après 18 heures d'incubation à 37° C

Les produits sont testés dans la gamme de concentrations de 0,125 à 256 mg/l (dilutions successives de raison 2).

Les concentrations minimales inhibitrices sont de l'ordre :

- de 0,25 $mg.ml^{-1}$ pour le Staphylocoque doré ;
- de 1 $mg.ml^{-1}$ pour les Entérocoques.

L'ensemble de ces études montre l'activité antibiotique intéressante du produit de l'invention à la fois par l'intensité de son activité ainsi que par l'étendue de son spectre d'action.

| EXEMPLE 24 : COMPOSITION PHARMACEUTIQUE : COMPRIME | |
|---|---|
| Comprimés dosés à 75 mg de déformyl - 19 formylméthyl - 19 tylosine<br>Formule de préparation pour 100 comprimés | |
| Déformyl - 19 formylméthyl - 19 tylosine ..... | 75 g |
| Amidon de blé ..... | 70 g |
| Amidon de maïs ..... | 60 g |
| Lactose ..... | 75 g |
| Stéarate de magnésium ..... | 9 g |
| Silice ..... | 4 g |
| Hydroxy propylcellulose ..... | 7 g |
| pour 100 comprimés. | |

**Revendications**

1/ Composés de formule générale (I) :

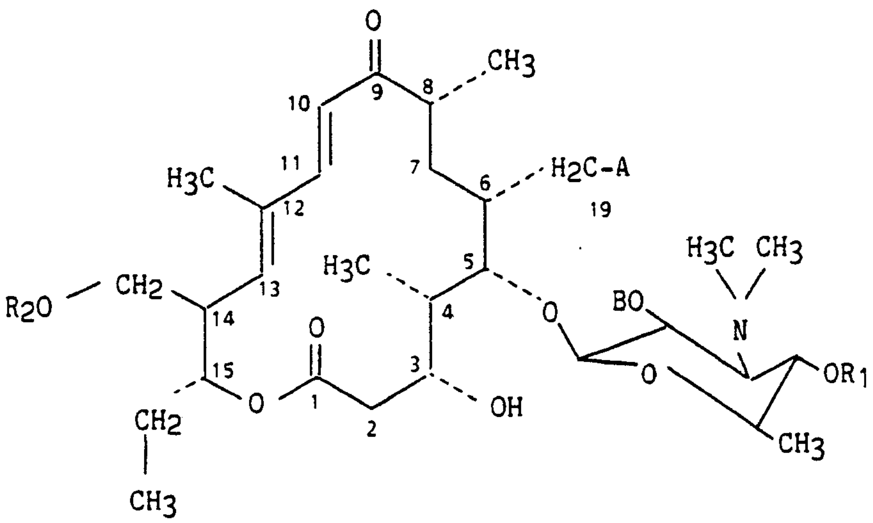

(I)

dans laquelle :

-$R_1$ représente un atome d'hydrogène ou un groupement :

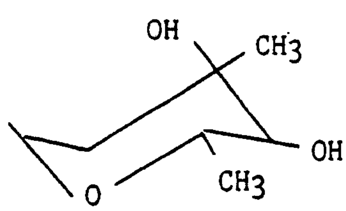

- $R_2$ représente un atome d'hydrogène ou un groupement :

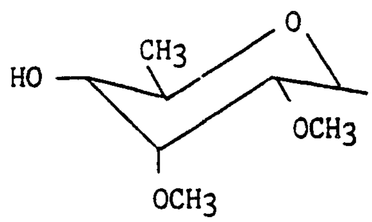

et dans le cas où $R_2$ représente un atome d'hydrogène, $R_1$ représente également un atome d'hydrogène.
- B représente un atome d'hydrogène ou un groupement acyle inférieur,
- A représente :
- un groupement $CH_2OH$ et dans ce cas B représente un groupement acyle inférieur, ou bien :
- un groupement $(CH_2)_nCN$,
- ou un groupement - $(CH_2)_nCHO$,
- ou un groupement - $(CH_2)_{(n+1)}OH$,
dans lequel n est un nombre entier compris entre 1 et 6, étant entendu que par groupement acyle inférieur, on entend un groupement comprenant de 1 à 5 atomes de carbone,
ainsi que leurs sels d'addition à un acide pharmaceutiquement acceptable.

2/ Composés selon la revendication 1 pour lesquels A représente un groupement $CH_2OH$ et B représente un groupement acyle inférieur et leurs sels d'addition à un acide pharmaceutiquement acceptable.

3/ Composés selon la revendication 1 pour lesquels A représente un groupement $(CH_2)_nCN$, n représentant un nombre entier compris entre 1 et 6 et leurs sels d'addition à un acide pharmaceutiquement acceptable.

4/ Composés selon la revendication 1 pour lesquels A représente un groupement $(CH_2)_nCHO$, n représentant un nombre entier compris entre 1 et 6 et leurs sels d'addition à un acide pharmaceutiquement acceptable.

5/ Composés selon la revendication 1 pour lesquels A représente un groupement $(CH_2)_{n+1}OH$, n représentant un nombre entier compris entre 1 et 6 et leurs sels d'addition à un acide pharmaceutiquement acceptable.

6/ Composé selon la revendication 1 qui est la cyanométhyl - 19 déformyl - 19 acétyl - 2′ tylosine et ses sels d'addition à un acide pharmaceutiquement acceptable.

7/ Composé selon l'une des revendications 1 ou 3 qui est la cyanométhyl - 19 déformyl - 19 tylosine et ses sels d'addition à un acide pharmaceutiquement acceptable.

8/ Composé selon l'une des revendications 1 ou 4 qui est la formylméthyl - 19 déformyl - 19 tylosine et ses sels d'addition à un acide pharmaceutiquement acceptable.

9/ Composé selon l'une des revendications 1 ou 5 qui est l'hydroxyméthyl - 19 déformyl - 19 acétyl - 2′ tylosine et ses sels d'addition à un acide pharmaceutiquement acceptable.

10/ Procédé de préparation des composés de formule (I) selon la revendication 1, caractérisé en ce que l'on fait réagir,
un dérivé de formule (II) :

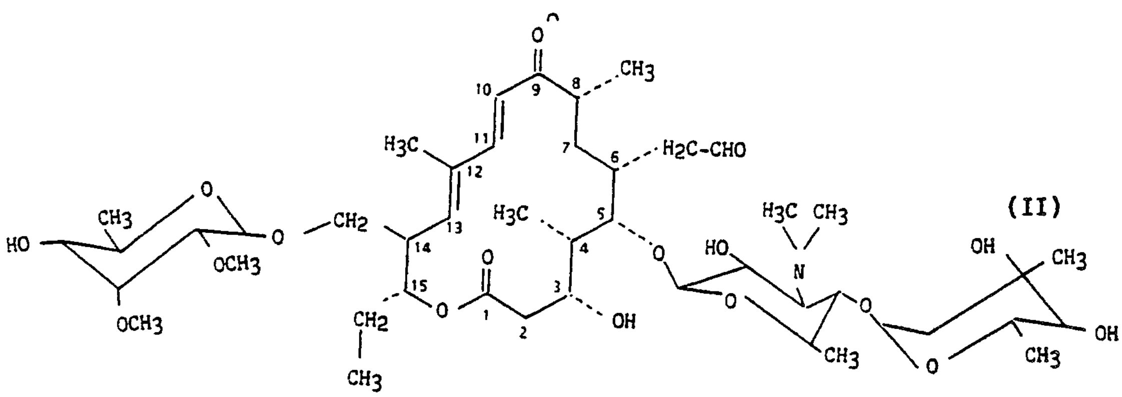

(II)

. que l'on soumet, lorsque dans le dérivé de formule (I) que l'on souhaite obtenir $R_1$ représente un atome d'hydrogène, à l'action de l'acide chlorhydrique dilué de normalité comprise entre 0,05 et 0,4 préférentiellement comprise entre 0,1 et 0,30 préférentiellement comprise entre 0,15 et 0,25, à température ambiante, pour conduire, après lavage par un solvant organique approprié, alcalinisation et extraction par un solvant organique approprié à un dérivé de formule (II/A) :

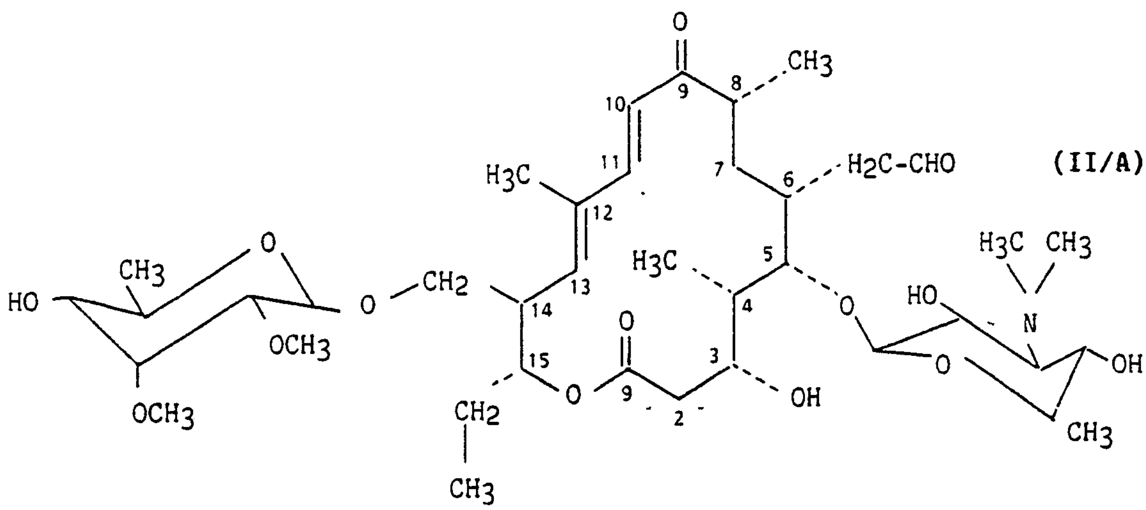

(II/A)

dérivé de formule (II/A) que l'on fait réagir, lorsque dans le dérivé de formule (I) que l'on souhaite obtenir $R_2$ représente un atome d'hydrogène, avec l'acide chlorhydrique de normalité comprise entre 0,25 et 0,75, préférentiellement entre 0,3 et 0,7, préférentiellement entre 0,4 et 0,6, à une température comprise préférentiellement entre 30 et 100° C ; préférentiellement comprise entre 50 et 90 ° C, préférentiellement entre 70 et 80 ° C, pour conduire, après lavage par un solvant organique approprié, alcalinisation et extraction par un solvant organique approprié à un dérivé de formule (II/B) :

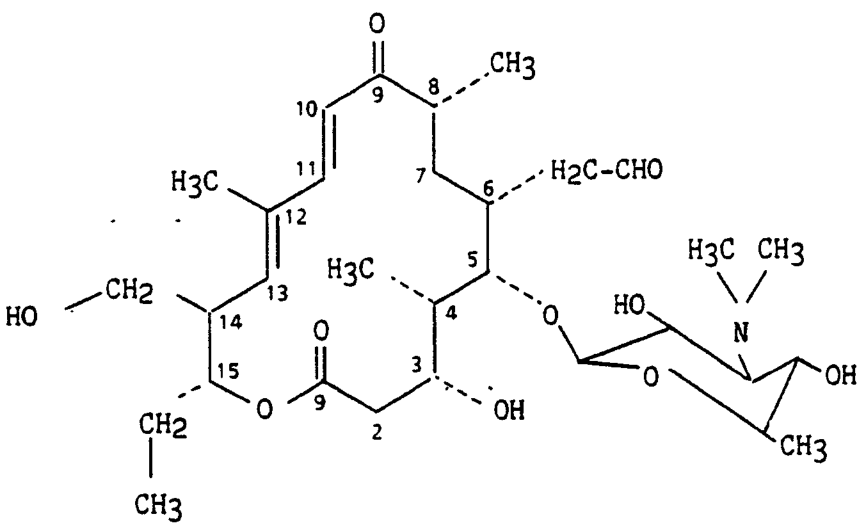

(II/B)

dérivé de formule (II), (II/A) ou (II/B), selon le dérivé de formule (I) que l'on souhaite obtenir, qui est soumis ou bien :

1/ à l'action d'un hydrure mixte de métal alcalin en milieu d'alcool aliphatique inférieur pour obtenir un dérivé de formule (III) :

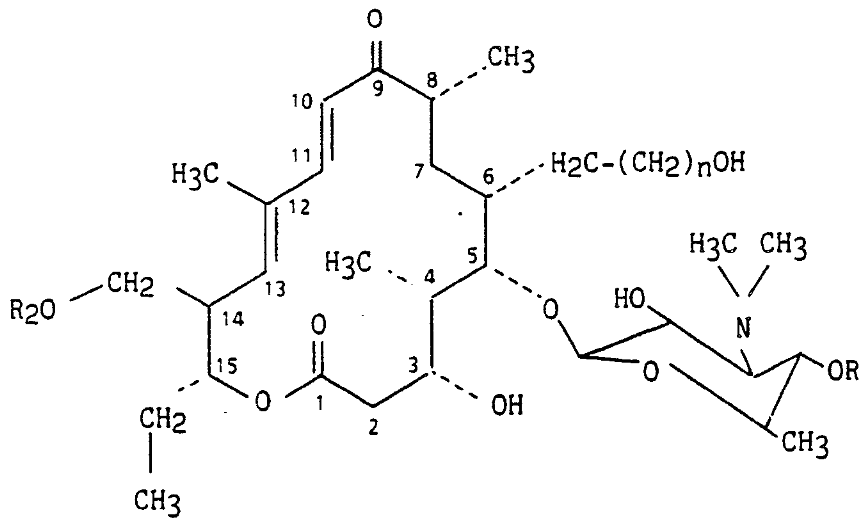

(III)

dans laquelle $R_1$ et $R_2$ ont la même signification que dans la formule (I), et n représente 1, que l'on soumet à l'action d'un anhydride d'acide carboxylique inférieur de formule Ac-OH dans laquelle Ac représente un groupement acyle inférieur, à température préférentiellement ambiante et préférentiellement sous atmosphère inerte pour obtenir un dérivé de formule (IV) :

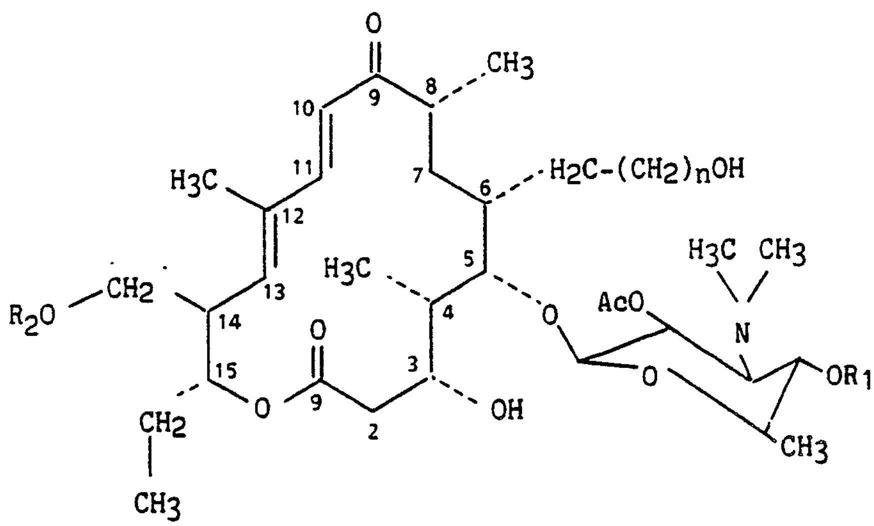

(IV)

dans laquelle Ac représente un groupement acyle inférieur, n vaut 1, $R_1$ et $R_2$ ont la même définition que dans la formule (I),
cas particulier de dérivés de formule (I) pour lesquels B représente un groupement acyle inférieur et A représente un groupement $CH_2OH$,
que l'on traite par le chlorure de tosyle, que l'on ajoute par petites fractions,
en présence de diméthylamino-4 pyridine pour obtenir après éventuelle purification, extraction et éventuelle chromatographie, un dérivé de formule (V) :

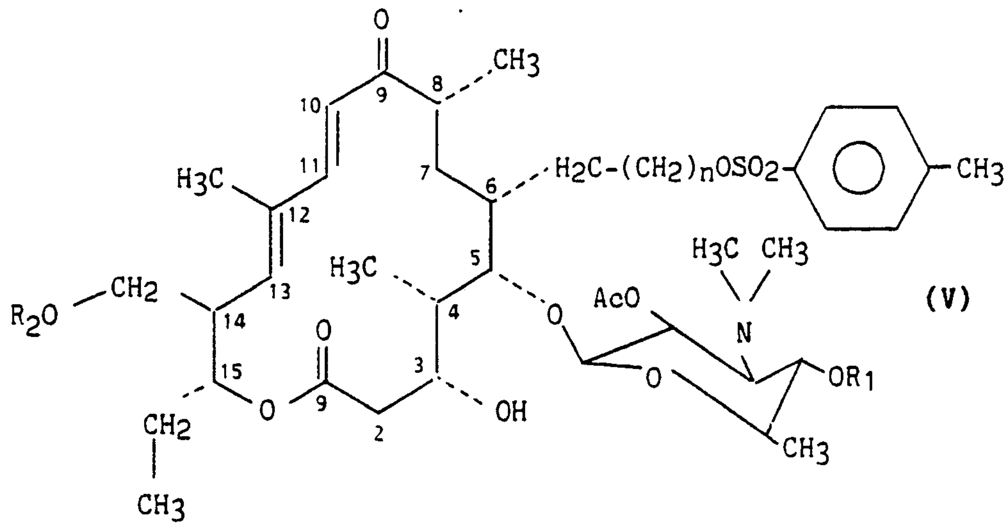

(V)

dans laquelle n, Ac, $R_1$ et $R_2$ ont la même signification que dans la formule (IV), que l'on traite par un cyanure de métal alcalin en présence d'un agent cryptant pour obtenir un dérivé de formule (VI) :

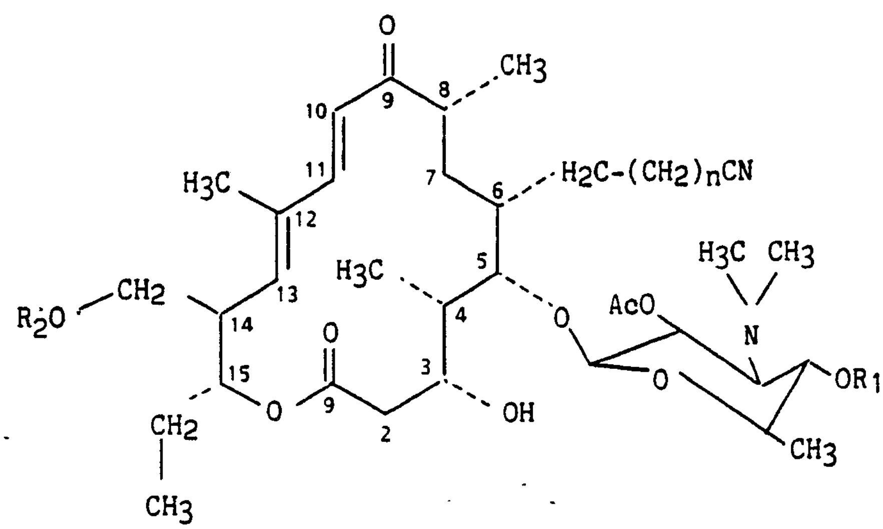

(VI)

dans laquelle n, Ac, $R_1$ et $R_2$ ont la même signification que dans la formule (IV),
cas particulier de dérivés de formule (I) pour lesquels B représente un groupement acyle inférieur et A représente un groupement $CH_2CN$,
que l'on soumet à action du méthanol,
pour obtenir un dérivé de formule (VII) :

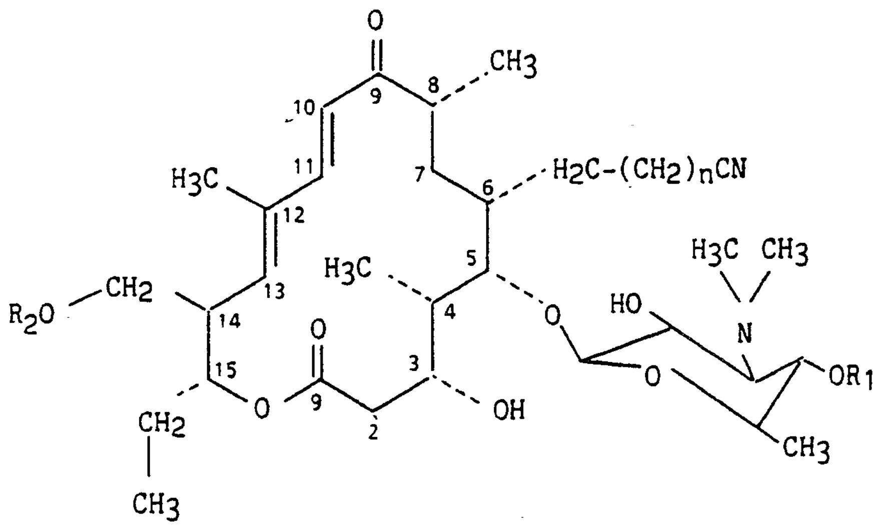

(VII)

dans laquelle $R_1$ et $R_2$ ont la même définition que dans la formule (I), et n représente 1, cas particulier des dérivés de formule (I) pour lesquels B représente un atome d'hydrogène et A représente un groupement $CH_2CN$, que l'on traite par le diisobutyl hydrure d'aluminium préférentiellement sous atmosphère inerte, pour conduire à un dérivé de formule (VIII) :

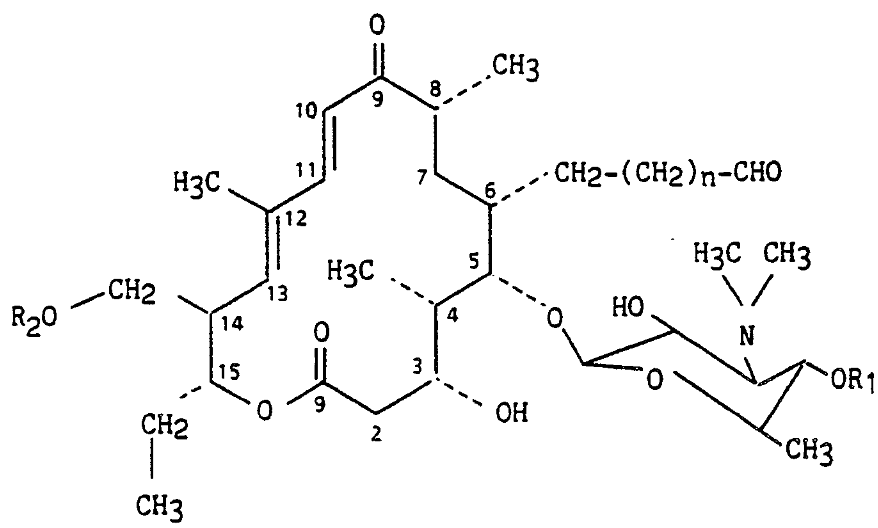

(VIII)

dans laquelle $R_1$ et $R_2$ ont la même définition que dans la formule (I), et n représente 1, cas particulier des dérivés de formule (I) pour lesquels B représente un atome d'hydrogène et A représente un groupement $CH_2$-CHO,

2/ou bien à l'action du chlorure de (paratoluène sulfonyle) méthyle en présence du n tert.butylate de métal alcalin dans un alcool aliphatique inférieur, pour conduire après éventuelle purification par chromatographie à un dérivé de formule (IX) :

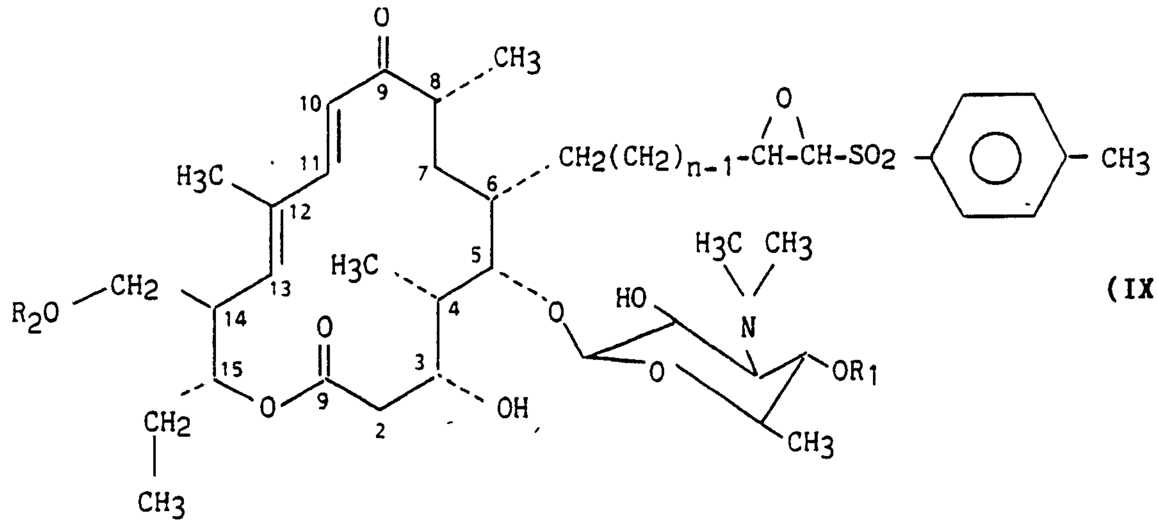

(IX)

dans laquelle $R_1$ et $R_2$ ont la même définition que dans la formule (I), et n représente 1, que l'on traite par un hydrure mixte de métal alcalin en milieu de diméthylformamide préférentiellement sous léger chauffage, pour conduire après éventuelle purification par chromatographie à un dérivé de formule (X) :

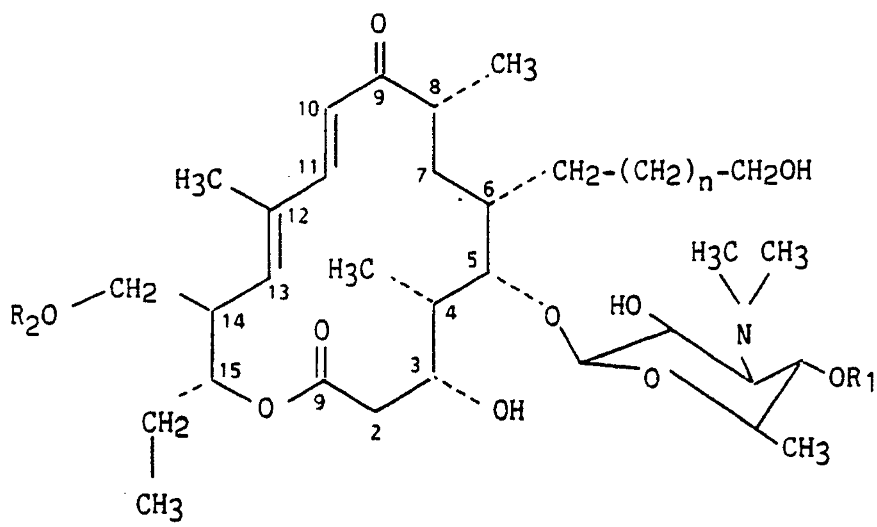

(X)

dans laquelle $R_1$ et $R_2$ ont la même définition que dans la formule (I), et n représente 1, cas particulier des dérivés de formule (I) pour lesquels A représente un groupement $(CH_2)_2$-OH et B représente un atome d'hydrogène, que l'on traite par le chlorochromate de pyridinium pour obtenir, après éventuelle purification par chromatographie, un produit de formule (VIII), lequel à son tour, si on le désire peut être soumis, comme indiqué plus haut, en fonction du dérivé de formule (I) que l'on souhaite obtenir,

- soit 3/ à l'action successive d'un hydrure mixte de métal alcalin d'anhydride d'acide carboxylique inférieur, de chlorure de tosyle en présence de dimétylamino - 4 pyridine, de cyanure de métal alcalin, de méthanol en présence d'un agent cryptant puis de diisobutyl hydrure d'aluminium, pour obtenir successivement les dérivés de formules (III/A), (IV/A), (V/A), (VI/A), (VII/A) et (VIII/A) cas particulier des dérivés de formules (III), (IV), (V), (VI), (VII) et (VIII), pour lesquels n représente 2, $R_1$ et $R_2$ ayant la même signification que dans la formule (I), Ac signifiant le cas échéant un groupement acyle inférieur,
- soit 4/ à l'action successive de chlorure de (paratoluène sulfonyle) méthyle en présence de tert.butylate de métal alcalin, puis d'hydrure mixte de métal alcalin en présence de diméthylformamide puis chlorochromate de pyridinium, pour conduire successivement aux dérivés de formules (IX/A), (X/A) et (VIII/A) cas particulier des dérivés de formules (IX), (X) et (VIII), pour lesquels n représente 2, $R_1$ et $R_2$ ayant la même signification que dans la formule (I),

dérivé de formule (VIII/A) qui à son tour est éventuellement soumis à une ou deux, trois ou quatre reprises à un traitement identique à ceux indiqués en 3/ ou 4/, pour conduire à un dérivés de formules (III/B), (IV/B), (V/B), (VI/B), (VII/B), (VIII/B), (IX/B), (X/B) cas particulier des dérivés de formules (III), (IV), (V), (VI), (VII), (VIII), (IX), (X) pour lesquels n représente 3, 4, 5 ou 6, selon que le traitement indiqué en 3/ ou 4/ est exécuté à 1, 2, 3 ou 4 reprises, R1 et R2 ayant la même signification que dans la formule (I) et Ac signifiant le cas échéant un groupement acyle inférieur,

dérivés que l'on purifie éventuellement par chromatographie sur colonne de silice à l'aide d'un mélange solvant approprié,

et que l'on peut, si on le désire salifier par un acide pharmaceutiquement acceptable.

11/ Composés de formule (V), (V/A) et (V/B) selon la revendication 10 utiles dans la préparation des composés de formule (I).

12/ Composés de formule (IX), (IX/A) et (IX/B) selon la revendication 10 utiles dans la préparation des composés de formule (I).

13/ Composition pharmaceutique contenant comme principe actif au moins un composé selon l'une des revendication 1 à 9 en combinaison avec un ou plusieurs excipients ou véhicules inertes, non toxiques, pharmaceutiquement acceptable.

14/ Composition pharmaceutique selon la revendication 13 contenant comme principe actif au moins un composé selon l'une des revendications 1 à 9 utilisables dans le traitement des maladies justifiant une antibiothérapie à large spectre.

Revendications pour l'Etat contractant suivant: ES

1/ Procédé de préparation de composés de formule générale (I) :

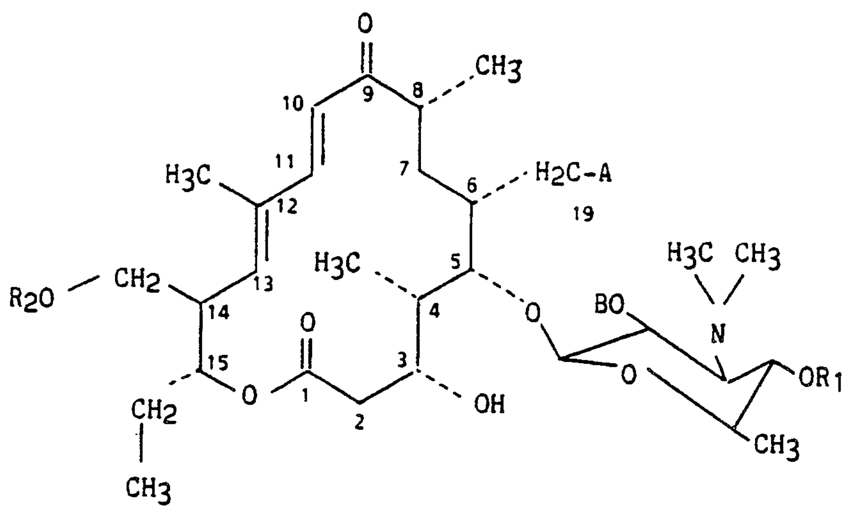

(I)

dans laquelle :

- $R_1$ représente un atome d'hydrogène ou un groupement :

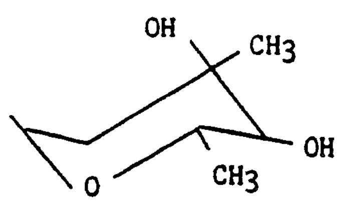

- $R_2$ représente un atome d'hydrogène ou un groupement :

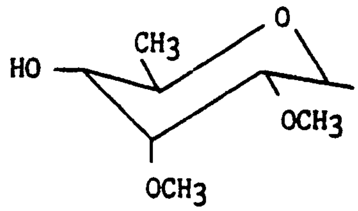

et dans le cas où $R_2$ représente un atome d'hydrogène, $R_1$ représente également un atome d'hydrogène.

- B représente un atome d'hydrogène ou un groupement acyle inférieur,
- A représente :
- un groupement $CH_2OH$ et dans ce cas B représente un groupement acyle inférieur, ou bien :
- un groupement $(CH_2)_nCN$,
- ou un groupement - $(CH_2)_nCHO$,
- ou un groupement - $(CH_2)_{(n+1)}OH$,

dans lequel n est un nombre entier compris entre 1 et 6, étant entendu que par groupement acyle inférieur, on entend un groupement comprenant de 1 à atomes de carbone,
ainsi que leurs sels d'addition à un acide pharmaceutiquement acceptable,
caractérisé en ce que l'on fait réagir,
un dérivé de formule (II) :

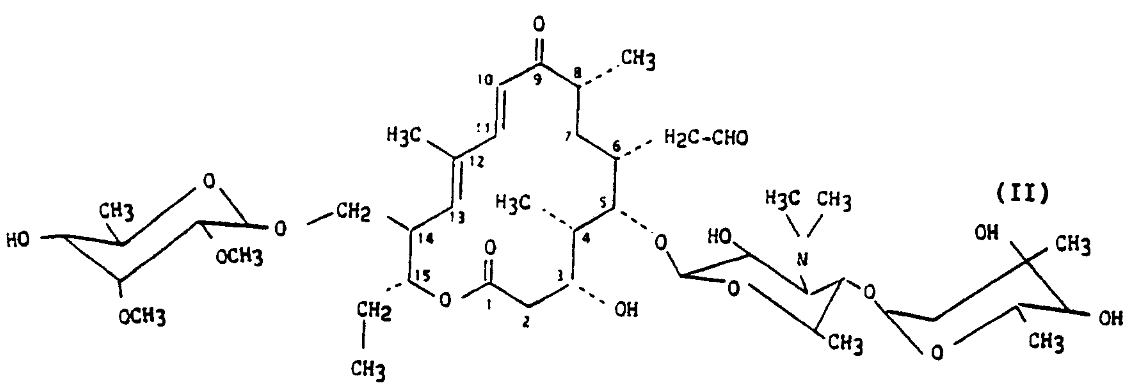

(II)

. que l'on soumet, lorsque dans le dérivé de formule (I) que l'on souhaite obtenir $R_1$ représente un atome d'hydrogène, à l'action de l'acide chlorhydrique dilué de normalité comprise entre 0,05 et 0,4 préférentiellement comprise entre 0,1 et 0,30 préférentiellement comprise entre 0,15 et 0,25, à température ambiante, pour conduire, après lavage par un solvant organique approprié, alcalinisation et extraction par un solvant organique approprié à un dérivé de formule (II/A) :

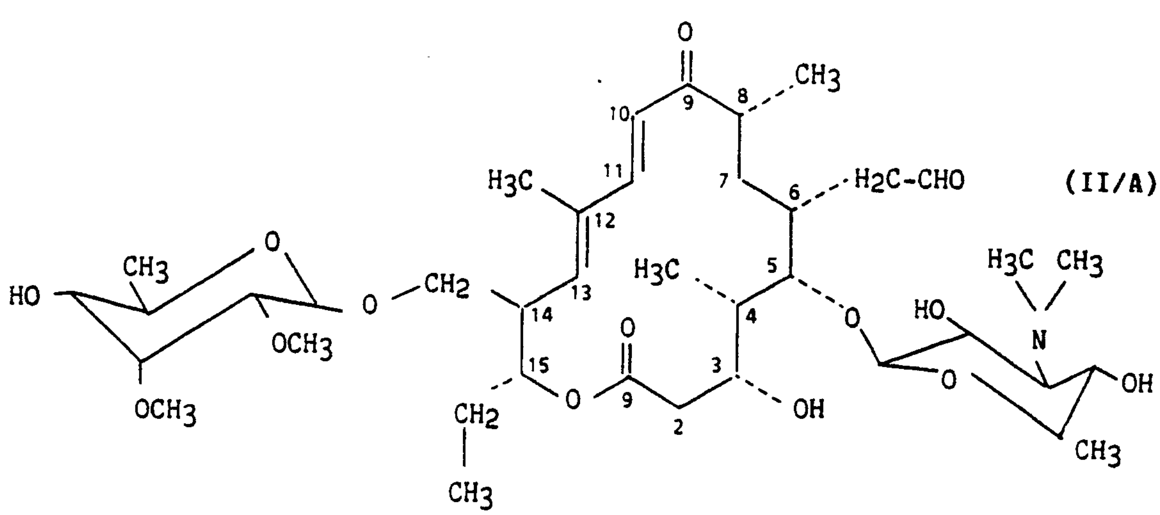

(II/A)

dérivé de formule (II/A) que l'on fait réagir, lorsque dans le dérivé de formule (I) que l'on souhaite obtenir $R_2$ représente un atome d'hydrogène, avec l'acide chlorhydrique de normalité comprise entre 0,25 et 0,75, préférentiellement entre 0,3 et 0,7, préférentiellement entre 0,4 et 0,6, à une température comprise préférentiellement entre 30 et 100° C ; préférentiellement comprise entre 50 et 90 ° C, préférentiellement entre 70 et 80° C, pour conduire, après lavage par un solvant organique approprié, alcalinisation et extraction par un solvant organique approprié à un dérivé de formule (II/B) :

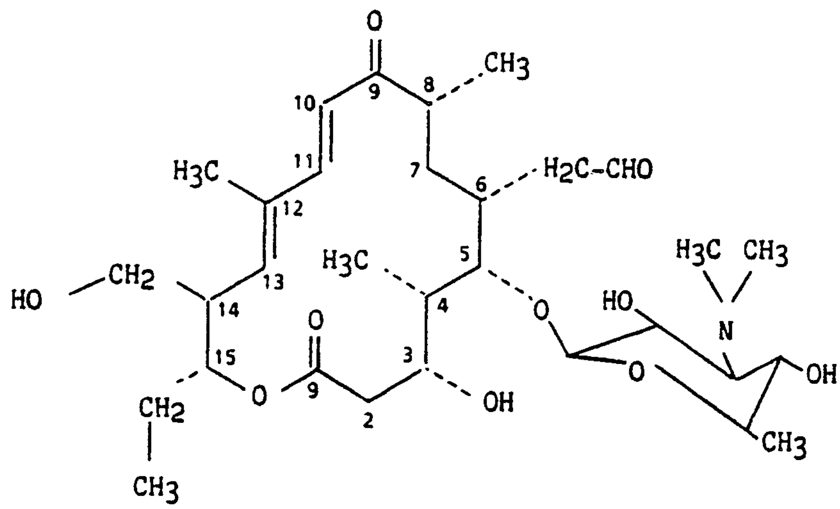

(II/B)

dérivé de formule (II), (II/A) ou (II/B), selon le dérivé de formule (I) que l'on souhaite obtenir, qui est soumis ou bien :

1/ à l'action d'un hydrure mixte de métal alcalin en milieu d'alcool aliphatique inférieur pour obtenir un dérivé de formule (III) :

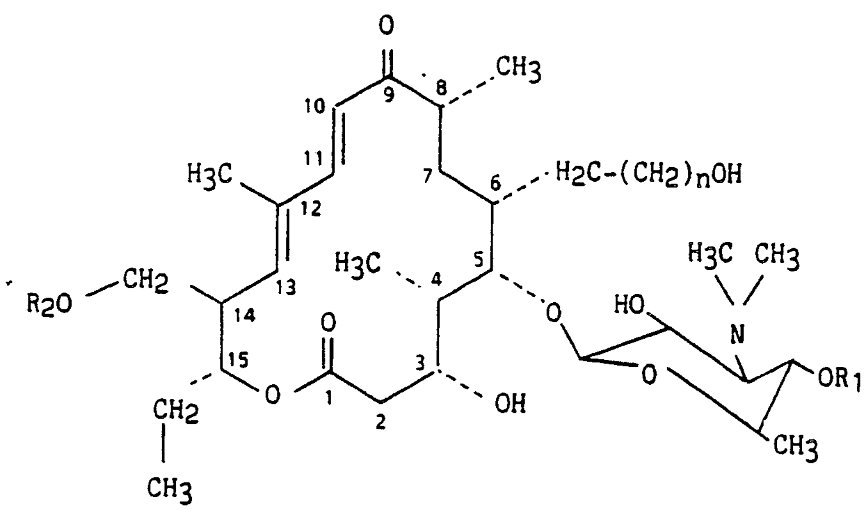

(III)

dans laquelle $R_1$ et $R_2$ ont la même signification que dans la formule (I), set n représente 1,

que l'on soumet à l'action d'un anhydride d'acide carboxylique inférieur de formule Ac-OH dans laquelle Ac représente un groupement acyle inférieur, à température préférentiellement ambiante et préférentiellement sous atmosphère inerte pour obtenir un dérivé de formule (IV) :

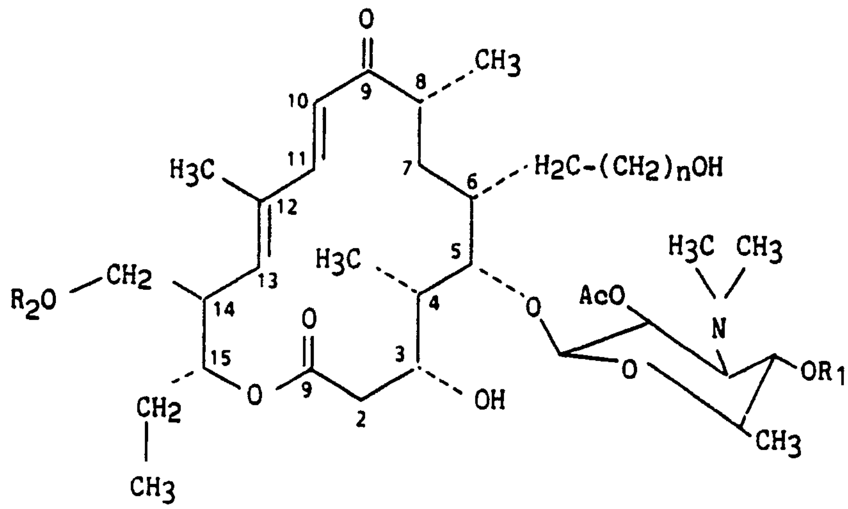

(IV)

dans laquelle Ac représente un groupement acyle inférieur, n vaut 1, $R_1$ et $R_2$ ont la même définition que dans la formule (I),
cas particulier de dérivés de formule (I) pour lesquels B représente un groupement acyle inférieur et A représente un groupement $CH_2OH$,
que l'on traite par le chlorure de tosyle, que l'on ajoute par petites fractions,
en présence de diméthylamino-4 pyridine pour obtenir après éventuelle purification, extraction et éventuelle chromatographie, un dérivé de formule (V) :

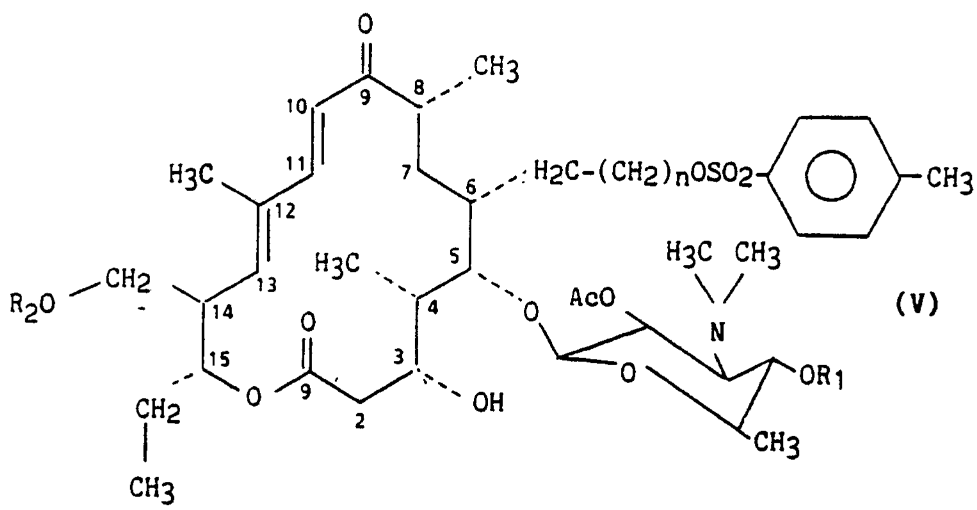

(V)

dans laquelle n, Ac, $R_1$ et $R_2$ ont la même signification que dans la formule (IV),
que l'on traite par un cyanure de métal alcalin en présence d'un agent cryptant pour obtenir un dérivé de formule (VI) :

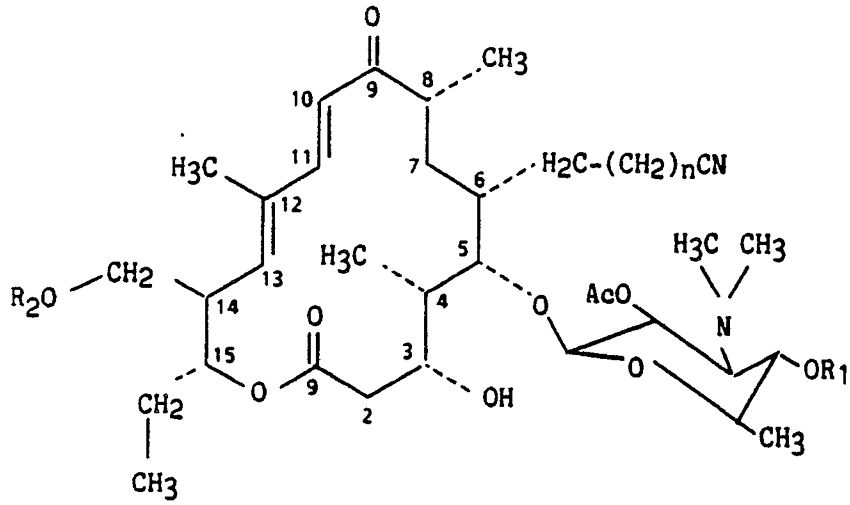

(VI)

dans laquelle n, Ac, $R_1$ et $R_2$ ont la même signification que dans la formule (IV), cas particulier de dérivés de formule (I) pour lesquels B représente un groupement acyle inférieur et A représente un groupement $CH_2CN$, que l'on soumet à action du méthanol, pour obtenir un dérivé de formule (VII) :

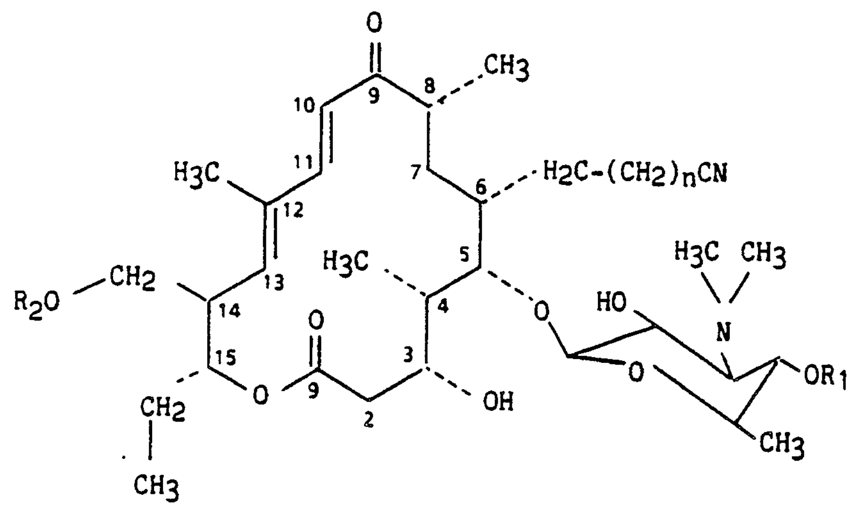

(VII)

dans laquelle $R_1$ et $R_2$ ont la même définition que dans la formule (I), et n représente 1, cas particulier des dérivés de formule (I) pour lesquels B représente un atome d'hydrogène et A représente un groupement $CH_2CN$, que l'on traite par le diisobutyl hydrure d'aluminium préférentiellement sous atmosphère inerte, pour conduire à un dérivé de formule (VIII) :

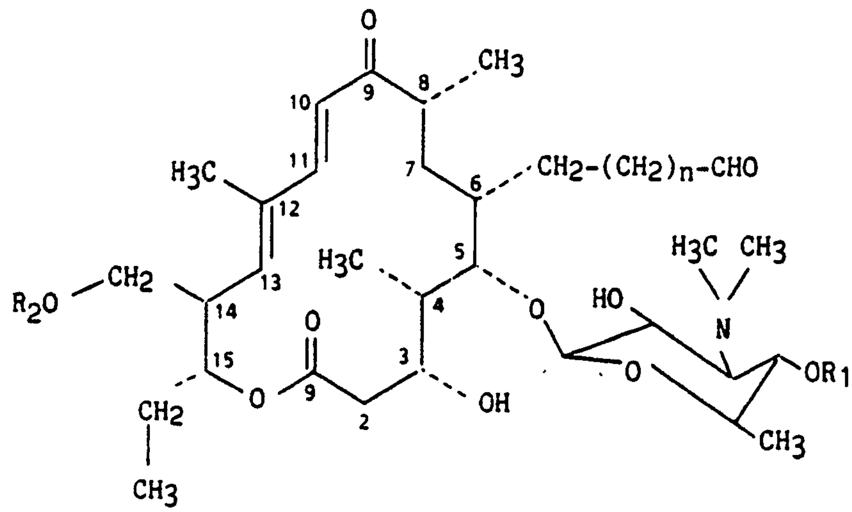

(VIII)

dans laquelle $R_1$ et $R_2$ ont la même définition que dans la formule (I), et n représente 1, cas particulier des dérivés de formule (I) pour lesquels B représente un atome d'hydrogène et A représente un groupement $CH_2$-CHO,

2/ ou bien à l'action du chlorure de (paratoluène sulfonyle) méthyle en présence du n tert.butylate de métal alcalin dans un alcool aliphatique inférieur, pour conduire après éventuelle purification par chromatographie à un dérivé de formule (IX) :

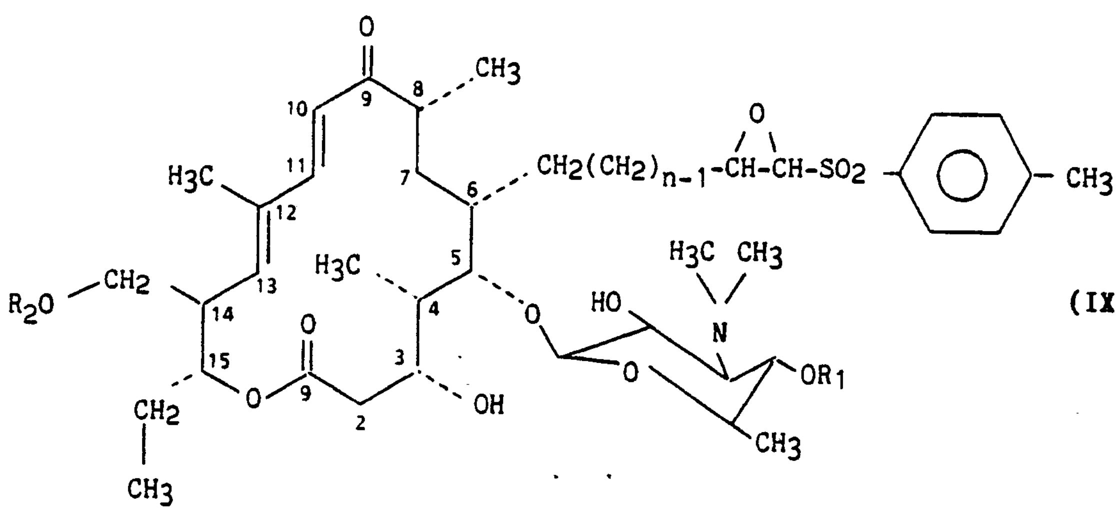

(IX)

dans laquelle $R_1$ et $R_2$ ont la même définition que dans la formule (I), et n représente 1, que l'on traite par un hydrure mixte de métal alcalin en milieu de diméthylformamide préférentiellement sous léger chauffage, pour conduire après éventuelle purification par chromatographie à un dérivé de formule (X) :

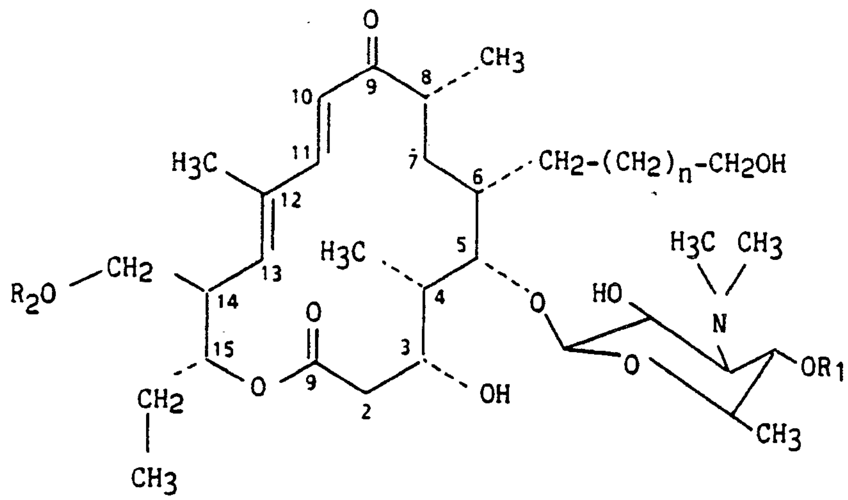

(X)

dans laquelle $R_1$ et $R_2$ ont la même définition que dans la formule (I), et n représente 1, cas particulier des dérivés de formule (I) pour lesquels A représente un groupement $(CH_2)_2$-OH et B représente un atome d'hydrogène,
que l'on traite par le chlorochromate de pyridinium pour obtenir, après éventuelle purification par chromatographie, un produit de formule (VIII),
lequel à son tour, si on le désire peut être soumis, comme indiqué plus haut, en fonction du dérivé de formule (I) que l'on souhaite obtenir,
- soit 3/ à l'action successive d'un hydrure mixte de métal alcalin d'anhydride d'acide carboxylique inférieur, de chlorure de tosyle en présence de dimétylamino - 4 pyridine, de cyanure de métal alcalin, de méthanol en présence d'un agent cryptant puis de diisobutyl hydrure d'aluminium, pour obtenir successivement les dérivés de formules (III/A), (IV/A), (V/A), (VI/A), (VII/A) et (VIII/A) cas particulier des dérivés de formules (III), (IV), (V), (VI), (VII) et (VIII), pour lesquels n représente 2, $R_1$ et $R_2$ ayant la même signification que dans la formule (I), Ac signifiant le cas échéant un groupement acyle inférieur,
- soit 4/ à l'action successive de chlorure de (paratoluène sulfonyle) méthyle en présence de tert.butylate de métal alcalin, puis d'hydrure mixte de métal alcalin en présence de diméthylfor mamide puis chlorochromate de pyridinium, pour conduire successivement aux dérivés de formules (IX/A), (X/A) et (VIII/A) cas particulier des dérivés de formules (IX), (X) et (VIII), pour lesquels n représente 2, $R_1$ et $R_2$ ayant la même signification que dans la formule (I),
dérivé de formule (VIII/A) qui à son tour est éventuellement soumis à une ou deux, trois ou quatre reprises à un traitement identique à ceux indiqués en 3/ ou 4/, pour conduire à un dérivés de formules (III/B), (IV/B), (V/B), (VI/B), (VII/B), (VIII/B), (IX/B), (X/B) cas particulier des dérivés de formules (III), (IV), (V), (VI), (VII), (VIII), (IX), (X) pour lesquels n représente 3, 4, 5 ou 6, selon que le traitement indiqué en 3/ ou 4/ est exécuté à 1, 2, 3 ou 4 reprises, R1 et R2 ayant la même signification que dans la formule (I) et Ac signifiant le cas échéant un groupement acyle inférieur,
dérivés que l'on purifie éventuellement par chromatographie sur colonne de silice à l'aide d'un mélange solvant approprié,
et que l'on peut, si on le désire salifier par un acide pharmaceutiquement acceptable.

2/ Procédé de préparation selon la revendication 1 de composés de formule générale (I) pour lesquels A représente un groupement $CH_2ON$ et B représente un groupement acyle inférieur et leurs sels d'addition à un acide pharmaceutiquement acceptable.

3/ Procédé de préparation selon la revendication 1 de composés de formule générale (I) pour lesquels A représente un groupement $(CH_2)_nCN$, n représentant un nombre entier compris entre 1 et 6 et leurs sels d'addition à un acide pharmaceutiquement acceptable.

4/ Procédé de préparation selon la revendication 1 de composés de formule générale (I) pour lesquels A représente un groupement $(CH_2)_nCHO$, n représentant un nombre entier compris entre 1 et 6 et leurs sels d'addition à un acide pharmaceutiquement acceptable.

5/ Procédé de préparation selon la revendication 1 de composés de formule générale (I) pour lesquels A représente un groupement $(CH_2)_{n+1}OH$, n représentant un nombre entier compris entre 1 et 6 et leurs sels d'addition à un acide pharmaceutiquement acceptable.

6/ Procédé de préparation selon l'une des revendications 1 ou 3 du composé de formule générale (I)

qui est la cyanométhyl - 19 déformyl -19 acétyl - 2′ tylosine et ses sels d'addition à un acide pharmaceutiquement acceptable.

7/ Procédé de préparation selon l'une des revendications 1 ou 3 du composé de formule (I) qui est la cyanométhyl - 19 déformyl - 19 tylosine et ses sels d'addition à un acide pharmaceutiquement acceptable.

8/ Procédé de préparation selon l'une des revendications 1 ou 4 du composé de formule (I) qui est la formylméthyl - 19 déformyl - 19 tylosine et ses sels d'addition à un acide pharmaceutiquement acceptable.

9/ Procédé selon l'une des revendications 1 ou 2 du composé de formule (I) qui est l'hydroxyméthyl - 19 déformyl - 19 acétyl - 2′ tylosine et ses sels d'addition à un acide pharmaceutiquement acceptable.

Revendications pour l'Etat contractant suivant: GR

1/ Procédé de préparation de composés de formule générale (I) :

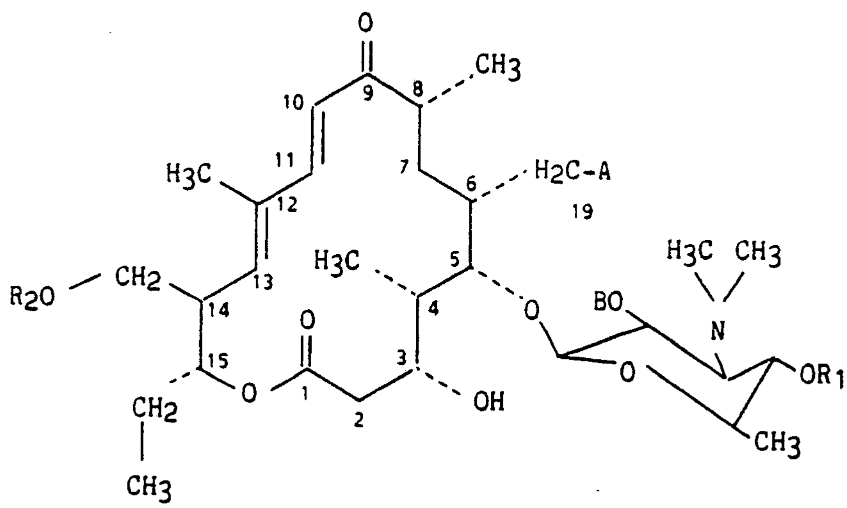

(I)

dans laquelle :

- $R_1$ représente un atome d'hydrogène ou un groupement :

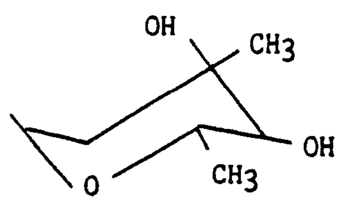

- $R_2$ représente un atome d'hydrogène ou un groupement :

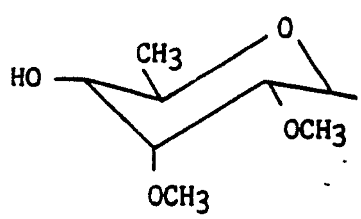

et dans le cas où $R_2$ représente un atome d'hydrogène, $R_1$ représente également un atome d'hydrogène.

- B représente un atome d'hydrogène ou un groupement acyle inférieur,
- A représente :
- un groupement $CH_2OH$ et dans ce cas B représente un groupement acyle inférieur, ou bien :
- un groupement $(CH_2)_nCN$,
- ou un groupement - $(CH_2)_nCHO$;.

- ou un groupement - $(CH_2)_{(n+1)}OH$,

dans lequel n est un nombre entier compris entre 1 et 6, étant entendu que par groupement acyle inférieur, on entend un groupement comprenant de 1 à atomes de carbone,

ainsi que leurs sels d'addition à un acide pharmaceutiquement acceptable,

caractérisé en ce que l'on fait réagir,

un dérivé de formule (II) :

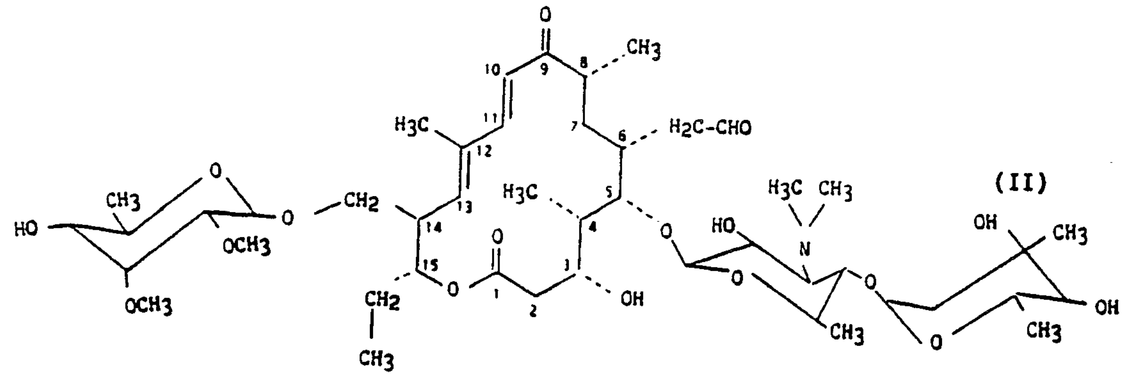

(II)

. que l'on soumet, lorsque dans le dérivé de formule (I) que l'on souhaite obtenir $R_1$ représente un atome d'hydrogène, à l'action de l'acide chlorhydrique dilué de normalité comprise entre 0,05 et 0,4 préférentiellement comprise entre 0,1 et 0,30 préférentiellement comprise entre 0,15 et 0,25, à température ambiante, pour conduire, après lavage par un solvant organique approprié, alcalinisation et extraction par un solvant organique approprié à un dérivé de formule (II/A) :

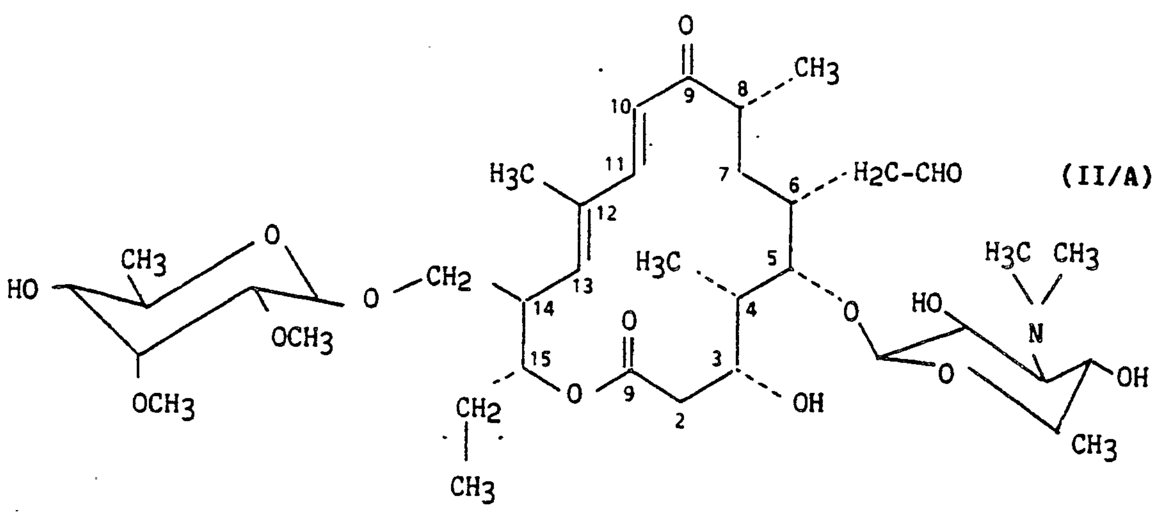

(II/A)

dérivé de formule (II/A) que l'on fait réagir, lorsque dans le dérivé de formule (I) que l'on souhaite obtenir $R_2$ représente un atome d'hydrogène, avec l'acide chlorhydrique de normalité comprise entre 0,25 et 0,75, préférentiellement entre 0,3 et 0,7, préférentiellement entre 0,4 et 0,6, à une température comprise préférentiellement entre 30 et 100 °C ; préférentiellement comprise entre 50 et 90 °C, préférentiellement entre 70 et 80 °C, pour conduire, après lavage par un solvant organique approprié, alcalinisation et extraction par un solvant organique approprié à un dérivé de formule (II/B) :

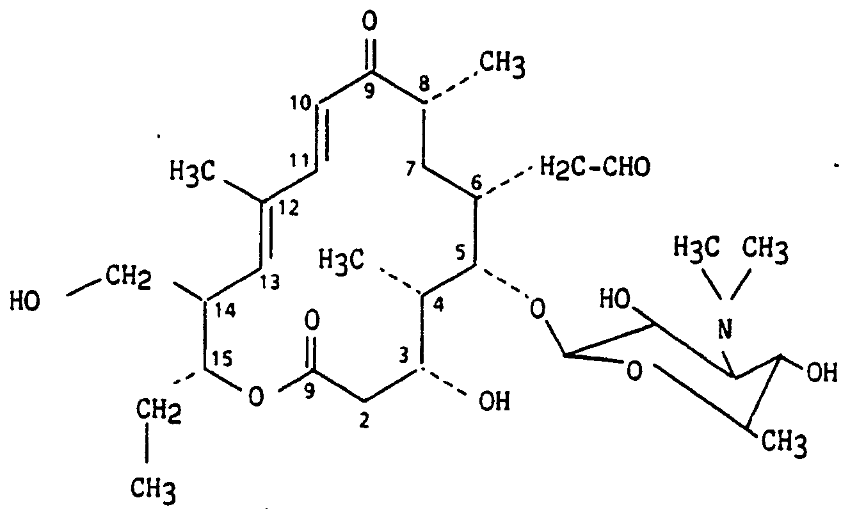

(II/B)

dérivé de formule (II), (II/A) ou (II/B), selon le dérivé de formule (I) que l'on souhaite obtenir, qui est soumis ou bien :

1/ à l'action d'un hydrure mixte de métal alcalin en milieu d'alcool aliphatique inférieur pour obtenir un dérivé de formule (III) :

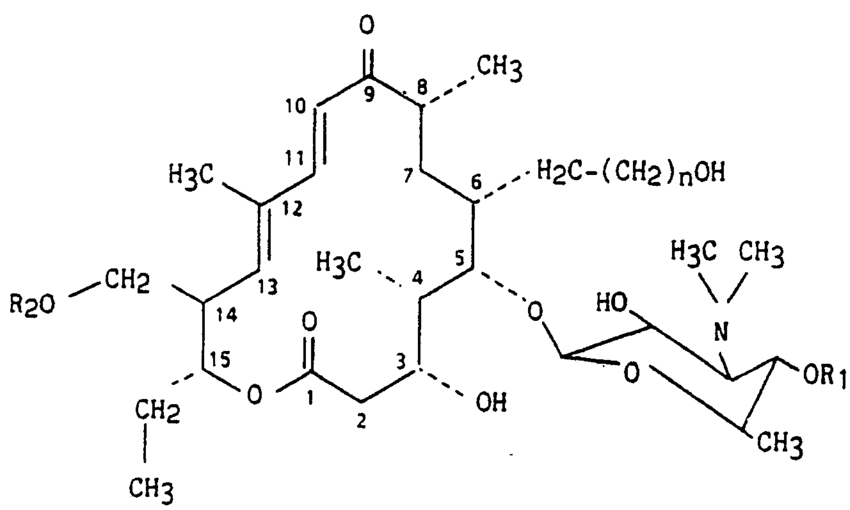

(III)

dans laquelle $R_1$ et $R_2$ ont la même signification que dans la formule (I), set n représente 1, que l'on soumet à l'action d'un anhydride d'acide carboxylique inférieur de formule Ac-OH dans laquelle Ac représente un groupement acyle inférieur, à température préférentiellement ambiante et préférentiellement sous atmosphère inerte pour obtenir un dérivé de formule (IV) :

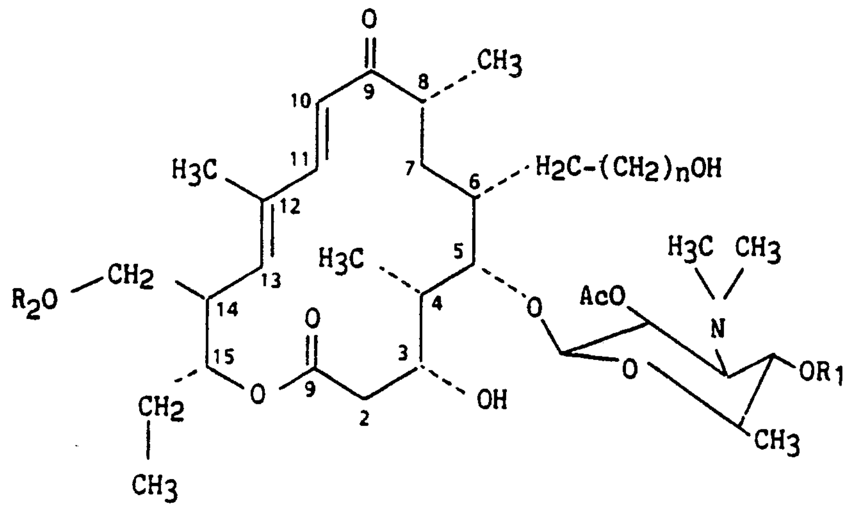

(IV)

dans laquelle Ac représente un groupement acyle inférieur, n vaut 1, $R_1$ et $R_2$ ont la même définition que dans la formule (I),
cas particulier de dérivés de formule (I) pour lesquels B représente un groupement acyle inférieur et A représente un groupement $CH_2OH$,
que l'on traite par le chlorure de tosyle, que l'on ajoute par petites fractions,
en présence de diméthylamino-4 pyridine pour obtenir après éventuelle purification, extraction et éventuelle chromatographie, un dérivé de formule (V) :

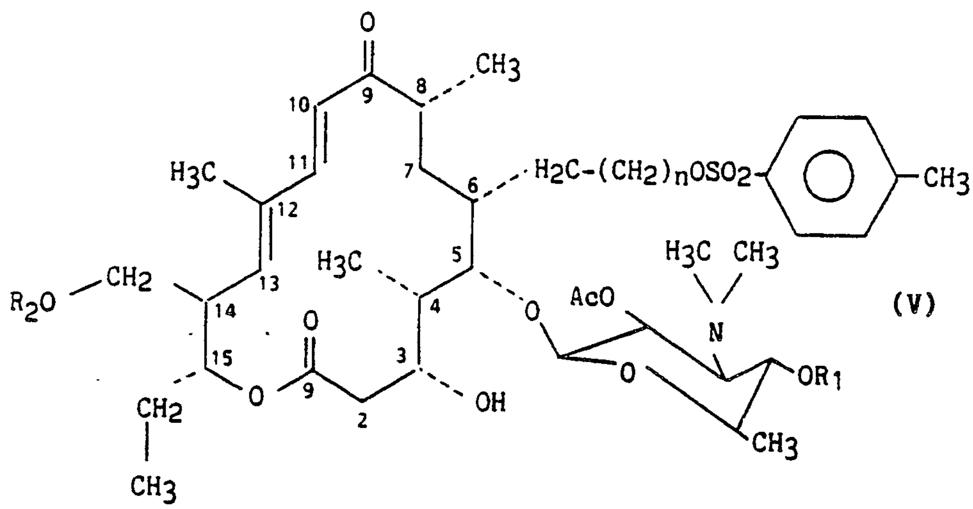

(V)

dans laquelle n, Ac, $R_1$ et $R_2$ ont la même signification que dans la formule (IV),
que l'on traite par un cyanure de métal alcalin en présence d'un agent cryptant pour obtenir un dérivé de formule (VI) :

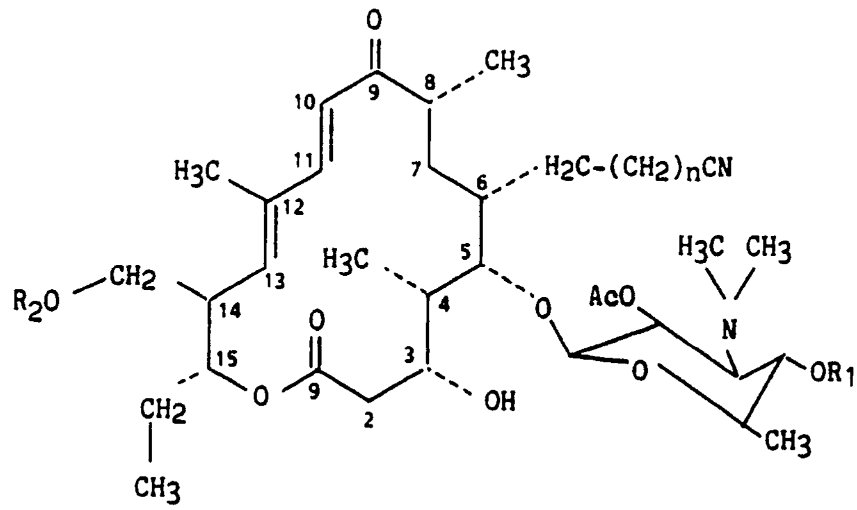

(VI)

dans laquelle n, Ac, $R_1$ et $R_2$ ont la même signification que dans la formule (IV), cas particulier de dérivés de formule (I) pour lesquels B représente un groupement acyle inférieur et A représente un groupement $CH_2CN$, que l'on soumet à action du méthanol, pour obtenir un dérivé de formule (VII) :

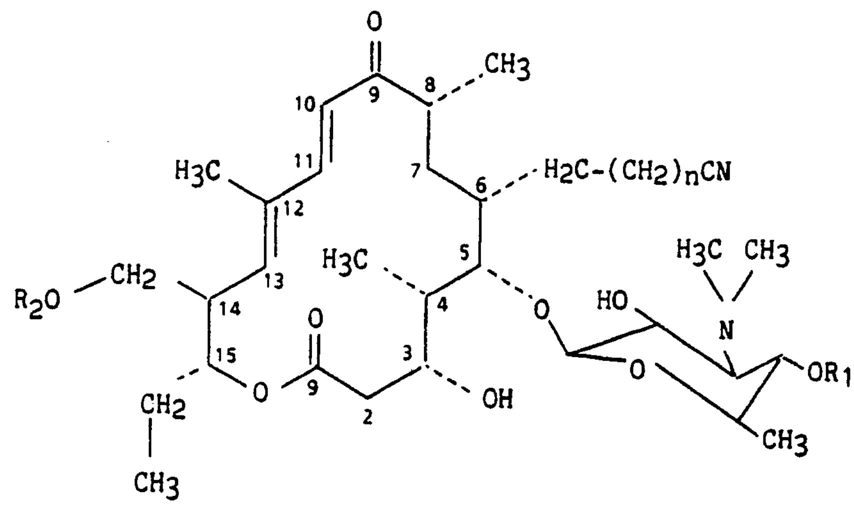

(VII)

dans laquelle $R_1$ et $R_2$ ont la même définition que dans la formule (I), et n représente 1, cas particulier des dérivés de formule (I) pour lesquels B représente un atome d'hydrogène et A représente un groupement $CH_2CN$, que l'on traite par le diisobutyl hydrure d'aluminium préférentiellement sous atmosphère inerte, pour conduire à un dérivé de formule (VIII) :

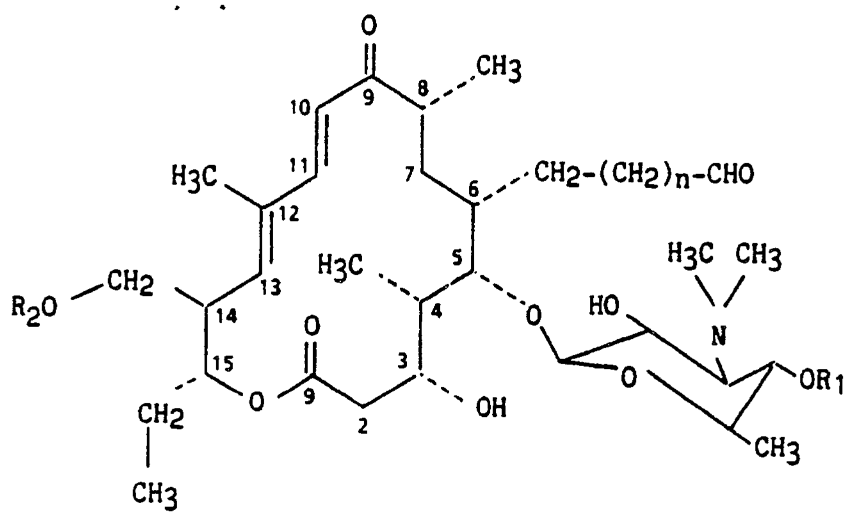

(VIII)

dans laquelle $R_1$ et $R_2$ ont la même définition que dans la formule (I), et n représente 1, cas particulier des dérivés de formule (I) pour lesquels B représente un atome d'hydrogène et A représente un groupement $CH_2$-CHO,

2/ ou bien à l'action du chlorure de (paratoluène sulfonyle) méthyle en présence du n tert.butylate de métal alcalin dans un alcool aliphatique inférieur, pour conduire après éventuelle purification par chromatographie à un dérivé de formule (IX) :

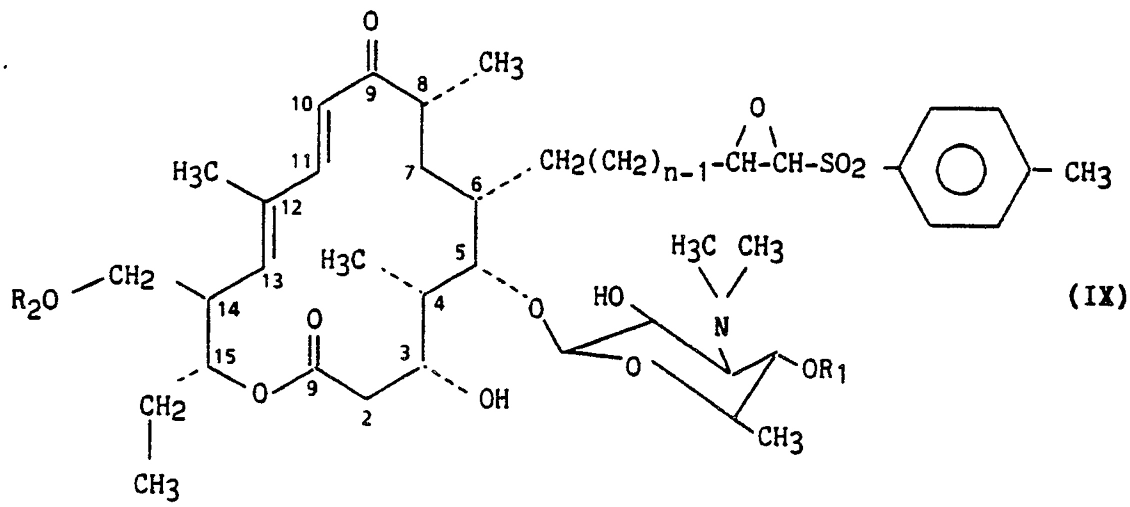

(IX)

dans laquelle $R_1$ et $R_2$ ont la même définition que dans la formule (I), et n représente 1, que l'on traite par un hydrure mixte de métal alcalin en milieu de diméthylformamide préférentiellement sous léger chauffage, pour conduire après éventuelle purification par chromatographie à un dérivé de formule (X) :

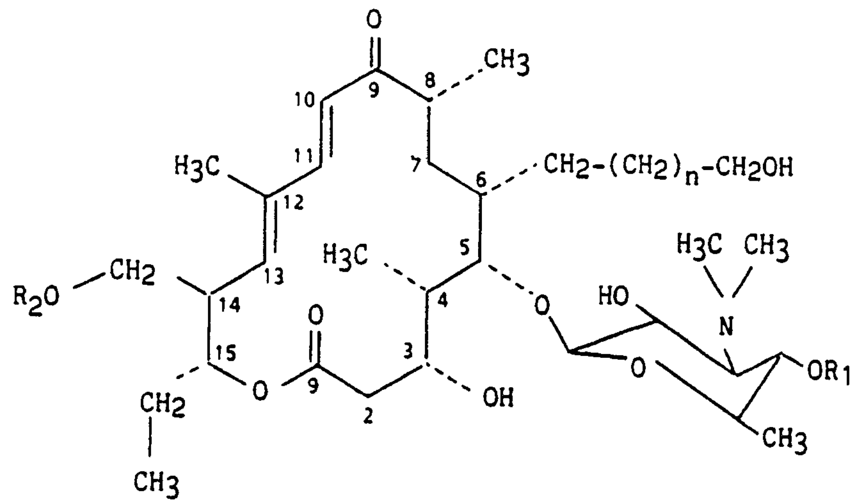

(X)

dans laquelle $R_1$ et $R_2$ ont la même définition que dans la formule (I), et n représente 1, cas particulier des dérivés de formule (I) pour lesquels A représente un groupement $(CH_2)_2$-OH et B représente un atome d'hydrogène,

que l'on traite par le chlorochromate de pyridinium pour obtenir, après éventuelle purification par chromatographie, un produit de formule (VIII),

lequel à son tour, si on le désire peut être soumis, comme indiqué plus haut, en fonction du dérivé de formule (I) que l'on souhaite obtenir,

- soit 3/ à l'action successive d'un hydrure mixte de métal alcalin d'anhydride d'acide carboxylique inférieur, de chlorure de tosyle en présence de dimétylamino - 4 pyridine, de cyanure de métal alcalin, de méthanol en présence d'un agent cryptant puis de diisobutyl hydrure d'aluminium, pour obtenir successivement les dérivés de formules (III/A), (IV/A), (V/A), (VI/A), (VII/A) et (VIII/A) cas particulier des dérivés de formules (III), (IV), (V), (VI), (VII) et (VIII), pour lesquels n représente 2, $R_1$ et $R_2$ ayant la même signification que dans la formule (I), Ac signifiant le cas échéant un groupement acyle inférieur,

- soit 4/ à l'action successive de chlorure de (paratoluène sulfonyle) méthyle en présence de tert.butylate de métal alcalin, puis d'hydrure mixte de métal alcalin en présence de diméthylfor mamide puis chlorochromate de pyridinium, pour conduire successivement aux dérivés de formules (IX/A), (X/A) et (VIII/A) cas particulier des dérivés de formules (IX), (X) et (VIII), pour lesquels n représente 2, $R_1$ et $R_2$ ayant la même signification que dans la formule (I),

dérivé de formule (VIII/A) qui à son tour est éventuellement soumis à une ou deux, trois ou quatre reprises à un traitement identique à ceux indiqués en 3/ ou 4/, pour conduire à un dérivés de formules (III/B), (IV/B), (V/B), (VI/B), (VII/B), (VIII/B), (IX/B), (X/B) cas particulier des dérivés de formules (III), (IV), (V), (VI), (VII), (VIII), (IX), (X) pour lesquels n représente 3, 4, 5 ou 6, selon que le traitement indiqué en 3/ ou 4/ est exécuté à 1, 2, 3 ou 4 reprises, R1 et R2 ayant la même signification que dans la formule (I) et Ac signifiant le cas échéant un groupement acyle inférieur,

dérivés que l'on purifie éventuellement par chromatographie sur colonne de silice à l'aide d'un mélange solvant approprié,

et que l'on peut, si on le désire salifier par un acide pharmaceutiquement acceptable.

2/ Procédé de préparation selon la revendication 1 de composés de formule générale (I) pour lesquels A représente un groupement $CH_2OH$ et B représente un groupement acyle inférieur et leurs sels d'addition à un acide pharmaceutiquement acceptable.

3/ Procédé de préparation selon la revendication 1 de composés de formule générale (I) pour lesquels A représente un groupement $(CH_2)_nCN$, n représentant un nombre entier compris entre 1 et 6 et leurs sels d'addition à un acide pharmaceutiquement acceptable.

4/ Procédé de préparation selon la revendication 1 de composés de formule générale (I) pour lesquels A représente un groupement $(CH_2)_nCHO$, n représentant un nombre entier compris entre 1 et 6 et leurs sels d'addition à un acide pharmaceutiquement acceptable.

5/ Procédé de préparation selon la revendication 1 de composés de formule générale (I) pour lesquels A représente un groupement $(CH_2)_{n+1}OH$, n représentant un nombre entier compris entre 1 et 6 et leurs sels d'addition à un acide pharmaceutiquement acceptable.

6/ Procédé de préparation selon l'une des revendications 1 ou 3 du composé de formule générale (I)

qui est la cyanométhyl - 19 déformyl -19 acétyl - 2′ tylosine et ses sels d'addition à un acide pharmaceutiquement acceptable.

7/ Procédé de préparation selon l'une des revendications 1 ou 3 du composé de formule (I) qui est la cyanométhyl - 19 déformyl - 19 tylosine et ses sels d'addition à un acide pharmaceutiquement acceptable.

8/ Procédé de préparation selon l'une des revendications 1 ou 4 du composé de formule (I) qui est la formylméthyl - 19 déformyl - 19 tylosine et ses sels d'addition à un acide pharmaceutiquement acceptable.

9/ Procédé selon l'une des revendications 1 ou 2 du composé de formule (I) qui est l'hydroxyméthyl - 19 déformyl - 19 acétyl - 2′ tylosine et ses sels d'addition à un acide pharmaceutiquement acceptable.

Office européen des brevets

# RAPPORT DE RECHERCHE EUROPEENNE

Numero de la demande

EP 90 40 1067

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 124 216 (ELI LILLY)<br>* Pages 32-51; revendications 1,7 * &<br>US-A-4 629 786 (Cat. D)<br>----- | 1,13 | C 07 H 17/08<br>A 61 K 31/71 |
| | | | **DOMAINES TECHNIQUES RECHERCHES (Int. Cl.5)** |
| | | | C 07 H 17/00<br>A 61 K 31/00 |

Le présent rapport a été établi pour toutes les revendications

| Lieu de la recherche | Date d'achèvement de la recherche | Examinateur |
|---|---|---|
| LA HAYE | 01-08-1990 | BRENNAN J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant